# EUROPEAN PATENT APPLICATION

(11) **EP 4 834 334 A1**
(43) Date of publication of application: **04.11.2026**
(21) Application number: 24913658.1
(22) Date of filing: 27.12.2024
(51) Int. Cl.: A61K 9/51, A61K 47/14, A61K 47/18, A61K 31/7105, A61K 48/00

(54) **NANOPARTICLE COMPOSITION FOR DRUG DELIVERY**

(30) Priority: 29.12.2023 KR 20230197400
(71) Applicant: SAMYANG BIOPHARM CORPORATION, Seongnam-si Gyeonggi-do 13488 (KR)
(72) Inventor: KIM, Yi Sak, Uijeongbu-si Gyeonggi-do 11708 (KR); KIM, Sol, Seongnam-si Gyeonggi-do 13488 (KR); PARK, Jong Min, Seongnam-si Gyeonggi-do 13590 (KR); LEE, So Jin, Seoul 02468 (KR); CHOI, Ji Hye, Seoul 05615 (KR)
(74) Representative: Gleiss Große Schrell und Partner mbB
(86) International application number: PCT/KR2024/021246
(87) International publication number: WO 2025/143853

(57) **Abstract**

The present invention relates to a composition for drug delivery, and a preparation method therefor, and, more specifically, to a composition for drug delivery, and a preparation method therefor, the composition having a form such that a drug is encapsulated inside a nanoparticle structure formed by a polymer and a cationic lipid with a specific structure.

## Description

### TECHNICAL FIELD

The present invention relates to a composition for drug delivery and a method for preparing the same, and more specifically, a composition for drug delivery which is in a form where a drug is encapsulated within a nanoparticle structure formed by a polymer and a cationic lipid having a specific structure, and a method for preparing the same.

### BACKGROUND ART

In therapies using anionic drugs including nucleic acid, technologies for safe and efficient drug delivery have been researched for a long time, and various carriers and techniques for delivery have been developed. Carriers are mainly divided into viral carriers utilizing adenovirus, retrovirus or the like, and non-viral carriers utilizing cationic lipid, cationic polymer or the like. Viral carriers are known as being exposed to risks such as non-specific immune response, etc. Thus, recent researches proceed in the direction to improve such disadvantages by using non-viral carriers. Although non-viral carriers are less efficient in comparison with viral carriers, they have advantages of fewer side effects in terms of *in vivo* safety.

The representative non-viral carriers for delivering nucleic acid material are a complex of cationic lipid and nucleic acid (lipoplex) and a complex of polycationic polymer and nucleic acid (polyplex). Such a cationic lipid or polycationic polymer stabilizes anionic drug by forming a complex through electrostatic interaction with the anionic drug and increases intracellular delivery, and for these reasons, various researches thereof have been conducted. However, when they are administered intravenously in an amount required to obtain a sufficient effect, severe toxicity is caused, although less toxic than viral carriers, resulting in that they are unsuitable for use in pharmaceutical products. Accordingly, there is a need to develop anionic drug delivery technology that is stable *in vivo* and capable of intracellular delivery to obtain sufficient effects, while reducing toxicity by minimizing the use of cationic polymer or cationic lipid that may cause toxicity.

Various anionic drug delivery compositions and preparation methods thereof have been disclosed, wherein a complex is formed by electrostatic interaction between nucleic acid and cationic lipid, and said complex is encapsulated within a nanoparticle structure of amphiphilic block copolymer. For example, Korean Laid-open Patent Publication No. 10-2017-0032858 discloses a composition for delivering an anionic drug, comprising the anionic drug as an active ingredient; a cationic compound; an amphiphilic block copolymer; and a salt of polylactic acid, wherein the anionic drug forms a complex with the cationic compound by electrostatic interaction, and the formed complex is encapsulated within the nanoparticle structure formed by the amphiphilic block copolymer and the salt of polylactic acid, and a method for preparing the same. However, including those disclosed in the above patent publication, the existing nanoparticle drug delivery systems still lack the efficiency to deliver drugs such as nucleic acid, polypeptide, or virus (especially mRNA) into the body.

### CONTENTS OF THE INVENTION

### PROBLEMS TO BE SOLVED

The purpose of the present invention is to provide a composition for drug delivery having significantly improved *in vivo* delivery efficiency of drugs such as nucleic acid, polypeptide, or virus (especially mRNA), as compared with previously known nanoparticle drug delivery systems, and a method for preparing the same.

### TECHNICAL MEANS

The first aspect of the present invention provides a composition for drug delivery comprising: effective ingredient selected from nucleic acid, polypeptide, virus or combination thereof; a lipid having a structure represented by the following Formula 1; and lipid-polymer, amphiphilic block copolymer, or a combination thereof: 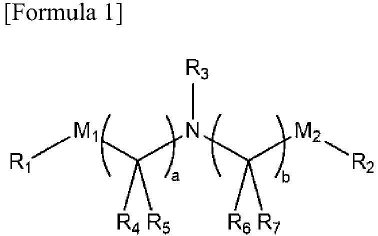 wherein, in the above Formula 1,
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₇ is independently hydrogen atom, or a substituted or unsubstituted, saturated or unsaturated hydrocarbon group, and
each of a and b is independently an integer of from 1 to 20.

The second aspect of the present invention provides a method for preparing a composition for drug delivery, comprising the steps of: (a) preparing a solution in which a lipid represented by the above Formula 1; and lipid-polymer, amphiphilic block copolymer, or a mixture thereof; are dissolved in a water-miscible organic solvent; and (b) to the solution prepared in step (a), adding effective ingredient selected from nucleic acid, polypeptide, virus, or combination thereof, and mixing them.

### EFFECT OF THE INVENTION

The composition for drug delivery according to the present invention can significantly improve *in vivo* delivery efficiency of drugs such as nucleic acid, polypeptide, or virus (especially mRNA), as compared with previously known nanoparticle drug delivery systems.

### CONCRETE MODE FOR CARRYING OUT THE INVENTION

The present invention will be explained in detail below.

### Effective ingredient

The effective ingredient comprised in the composition for drug delivery of the present invention is selected from nucleic acid, polypeptide, virus, or combination thereof.

The "nucleic acid" may be, for example, DNA, RNA, siRNA, shRNA, miRNA, mRNA, aptamer, antisense oligonucleotide, or a combination thereof, but it is not limited thereto.

The "polypeptide" may mean a protein having activity in the body such as antibody or fragment thereof, cytokine, hormone or analog thereof, or a protein that can be recognized as antigen through a series of processes in the body, including polypeptide sequence of antigen, analog or precursor thereof.

The "virus" may be an oncolytic virus and, for example, may be one or more selected from the group consisting of adenovirus, AAV, vaccinia virus, herpes simplex virus (HSV), and vesicular stomatitis virus (VSV). In an embodiment, the oncolytic virus is an adenovirus. The adenovirus used in an embodiment of the present invention contains a luciferase gene, which can be confirmed through imaging.

The virus can express various types of therapeutic genes within the body of an individual and is not limited to specific molecular weight, protein, bioactivity or therapeutic field. The prophylactic virus can induce immunity within the body of an individual against a target disease. A composition containing a prophylactic virus to disease has the advantage of reducing immunity induction by the virus itself, capability of designating or expanding target cells, and reducing the hyperimmune response to the virus upon re-administration, thereby enabling effective effects to be obtained through multiple inoculations.

In an embodiment, the effective ingredient is mRNA (messenger RNA).

The mRNA may be chemically modified in its backbone, sugar, or base, or may be terminally modified, for purposes such as increasing stability in the bloodstream or reducing an immune response.

In an embodiment, the amount of the effective ingredient may be, based on the dry weight of the total composition, 0.05 wt% or more, 0.1 wt% or more, 0.2 wt% or more, 0.3 wt% or more, 0.4 wt% or more, or 0.5 wt% or more, and it may also be 10 wt% or less, 9 wt% or less, 8 wt% or less, 7 wt% or less, 6 wt% or less, 5 wt% or less, 4 wt% or less, or 3 wt% or less. If the amount of the effective ingredient is too less, the amount of delivery carrier becomes too much as compared with the drug, and thus there may be a side effect due to the delivery carrier. To the contrary, if the amount of the effective ingredient is too much, the amount of drug not encapsulated in nanoparticles becomes too much, and thus the efficiency decreases.

### Lipid

The lipid comprised in the nanoparticle composition of the present invention is has a structure represented by the following Formula 1: 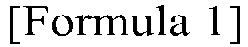 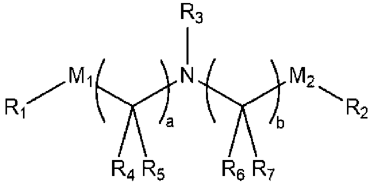 wherein, in the above Formula 1,
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₇ is independently hydrogen atom, or a substituted or unsubstituted, saturated or unsaturated hydrocarbon group, and
each of a and b is independently an integer of from 1 to 20.

The scope of the lipid comprised in the composition for drug delivery of the present invention includes not only those having the structure of the above Formula 1 but also cationic forms thereof.

As used herein, the expression "substituted or unsubstituted" for any group means that, unless specified otherwise, the group is not substituted, or is substituted with one or more substituents selected from -OH, halogen atom, C₁₋₈ alkyl group (more specifically, C₃₋₇ alkyl group) or C₁₋₈ halogenated alkyl group (more specifically, C₃₋₇ halogenated alkyl group).

According to an embodiment, in the above Formula 1, each of M₁ and M₂ may be independently selected from the group consisting of -C(O)O-, -OC(O)-, -OC(O)-M'-C(O)O-, - C(O)N(R')-, -N(R')C(O)-, -C(O)-, -C(S)-, -C(S)S-, -SC(S)-, -CH(OH)-, -P(O)(OR')O-, -S(O)₂-, - S-S-, arylene (more specifically C₆₋₂₀ arylene, still more specifically C₆₋₁₀ arylene), and heteroarylene (more specifically C₃₋₂₀ heteroarylene, still more specifically C₃₋₁₀ heteroarylene, having one or more (e.g., 1 to 3) heteroatoms selected from N, O and S), wherein M' may be a direct bond, C₁₋₁₃ alkylene (more specifically C₁₋₆ alkylene) or C₂₋₁₃ alkenylene (more specifically C₂₋₆ alkenylene), and each R' may be independently selected from the group consisting of hydrogen atom, C₁₋₁₈ alkyl (more specifically C₁₋₁₀ alkyl, still more specifically C₁₋₆ alkyl) and C₂₋₁₈ alkenyl (more specifically C₂₋₁₀ alkenyl, still more specifically C₂₋₆ alkenyl).

According to an embodiment, in the above Formula 1, each of R₁ and R₂ may be independently selected from the group consisting of substituted or unsubstituted C₃₋₂₀ cycloalkyl (more specifically C₃₋₁₅ cycloalkyl, still more specifically C₆₋₁₅ cycloalkyl), substituted or unsubstituted C₃₋₂₀ cycloalkenyl (more specifically C₃₋₁₅ cycloalkenyl, still more specifically C₆₋₁₅ cycloalkenyl), substituted or unsubstituted C₆₋₂₀ aryl (more specifically C₆₋₁₄ aryl), substituted or unsubstituted C₃₋₂₀ heterocycloalkyl (more specifically C₃₋₁₅ heterocycloalkyl), substituted or unsubstituted C₃₋₂₀ heterocycloalkenyl (more specifically C₃₋₁₅ heterocycloalkenyl), and substituted or unsubstituted C₃₋₂₀ heteroaryl (more specifically C₃₋₁₅ heteroaryl), wherein each of the heterocycloalkyl, heterocycloalkenyl and heteroaryl may independently have one or more (e.g., 1 to 3) heteroatoms selected from N, O and S.

According to an embodiment, in the above Formula 1, R₃ may be selected from the group consisting of hydrogen atom, substituted or unsubstituted C₁₋₆ alkyl, substituted or unsubstituted C₃₋₆ carbocyclic group, -(CH₂)ₙQ, - (CH₂)ₙCHQR, -CHQR and -CQ(R)₂, wherein each R may be independently selected from the group consisting of hydrogen atom, C₁₋₃ alkyl and C₂₋₃ alkenyl; Q may be selected from the group consisting of carbocyclic group, heterocyclic group, -OR, - O(CH₂)ₙN(R)₂, -C(O)OR, -OC(O)R, -CX₃, -CX₂H, -CXH₂, -CN, -N(R)₂, -C(O)N(R)₂, - N(R)C(O)R, -N(R)S(O)₂R, -N(R)C(O)N(R)₂, -N(R)C(S)N(R)₂, -N(R)R₁₂, N(R)S(O)₂R₁₂, - O(CH₂)ₙOR, -N(R)C(=NR₁₃)N(R)₂, -N(R)C(=CHR₁₃)N(R)₂, -OC(O)N(R)₂, -N(R)C(O)OR, - N(OR)C(O)R, -N(OR)S(O)₂R, -N(OR)C(O)OR, -N(OR)C(O)N(R)₂, -N(OR)C(S)N(R)₂, - N(OR)C(=NR₁₃)N(R)₂, -N(OR)C(=CHR₁₃)N(R)₂, -C(=NR₁₃)N(R)₂, - C(=NR₁₃)R, -C(O)N(R)OR and -C(R)N(R)₂C(O)OR, wherein each n is independently an integer of from 1 to 5; R₁₂ is selected from the group consisting of C₃₋₆ carbocyclic group and heterocyclic group; R₁₃ is selected from the group consisting of H, CN, NO₂, C₁₋₆ alkyl, -OR, -S(O)₂R, -S(O)₂N(R)₂, C₂₋₆ alkenyl, C₃₋₆ carbocyclic group and heterocyclic group; each R is independently selected from the group consisting of hydrogen atom, C₁₋₃ alkyl and C₂₋₃ alkenyl; each X is independently selected from the group consisting of F, CI, Br and I, provided that when R₃ is -(CH₂)ₙQ, -(CH₂)ₙCHQR, -CHQR or -CQ(R)₂, (i) if n is 1, 2, 3, 4, or 5, then Q is not -N(R)₂, or (ii) if n is 1 or 2, Q is not 5-, 6- or 7-membered heterocycloalkyl.

According to an embodiment, in the above Formula 1, each of R₄ to R₇ may be independently selected from the group consisting of hydrogen atom, C₁₋₃ alkyl and C₂₋₃ alkenyl.

According to an embodiment, in the above Formula 1, each of a and b may be independently an integer of from 1 to 15, and even more concretely, an integer of from 3 to 13.

Still more specifically, in the above Formula 1, each of M₁ and M₂ may be independently selected from the group consisting of -C(O)O-, -OC(O)-, -C(O)N(R')- and -N(R')C(O)-, wherein R' is the same as defined above.

Still more specifically, in the above Formula 1, each of R₁ and R₂ may be independently substituted or unsubstituted C₃₋₁₅ cycloalkyl.

Still more specifically, in the above Formula 1, R₃ may be hydrogen atom, or substituted or unsubstituted C₁₋₃ alkyl.

Still more specifically, in the above Formula 1, R₄ to R₇ may be hydrogen atom.

Still more specifically, in the above Formula 1, each of a and b may be independently an integer of from 5 to 11, and even more specifically, an integer of from 5 to 9.

In an embodiment, R₁ and R₂ are different from each other, wherein R₁ may be substituted or unsubstituted C₆₋₁₅ cycloalkyl, and R₂ may be substituted or unsubstituted C₃₋₆ cycloalkyl.

Even more specifically, the above lipid may be one having a structure selected from the following Formulas A to R:

| Formula | Structure | Formula | Structure |
|---|---|---|---|
| A | 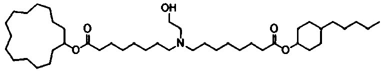 | J | 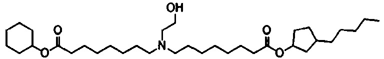 |
| B | 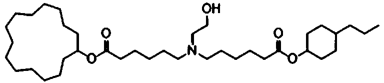 | K | 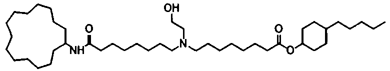 |
| C | 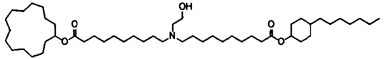 | L | 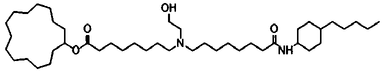 |
| D | 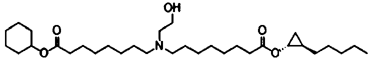 | M | 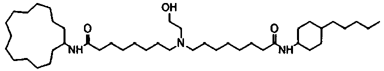 |
| E | 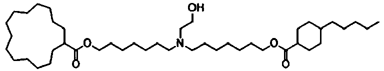 | N | 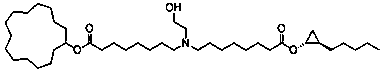 |
| F | 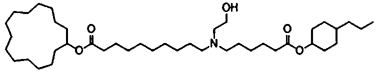 | O | 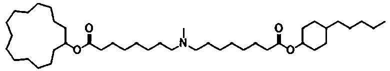 |
| G | 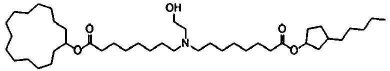 | P | 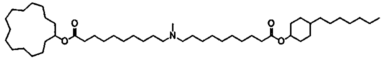 |
| H | 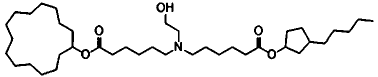 | Q | 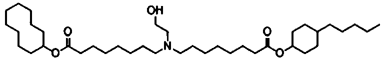 |
| I | 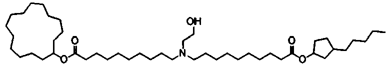 | R | 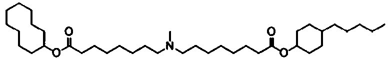 |

In an embodiment, the amount of the lipid in the composition for drug delivery of the present invention may be, based on the dry weight of the total composition, 1 wt% or more, 3 wt% or more, 5 wt% or more, 7 wt% or more, or 9 wt% or more, and it may also be 95 wt% or less, 90 wt% or less, 85 wt% or less, 80 wt% or less, or 75 wt% or less. If the amount of the lipid is too less, it may not be sufficient to form nanoparticles. To the contrary, if the amount of the lipid is too much, the size of the nanoparticle becomes too large, and thus the nanoparticle stability may be lowered and the rate of loss during filter sterilization may increase.

The lipid having the structure represented by Formula 1 may serve both as an ionizable lipid and as a helper lipid in the composition for drug delivery. The term "ionizable lipid" refers to a lipid capable of modulating its charge depending on pH and is one of the key components of lipid nanoparticle compositions useful for delivering nucleic acids such as RNA, DNA, etc. The term "helper lipid" refers to a lipid that enhances the particle stability and fluidity of lipid nanoparticles and may encompass, in addition to the ionizable lipid, various types of lipids such as phospholipids included in lipid nanoparticles. However, even though the lipid having the structure represented by Formula 1 may perform the functions of both an ionizable lipid and a helper lipid, the use of such additional lipid components is not excluded from the present invention. Accordingly, the composition for drug delivery of the present invention may further comprise, as needed, one or more ionizable lipids other than the lipid having the structure represented by Formula 1 or one or more helper lipids other than the lipid having the structure represented by Formula 1.

### Polymer

The composition for drug delivery of the present invention comprises lipid-polymer, amphiphilic block copolymer, or a combination thereof, as polymer component.

The lipid-polymer is a polymer having both hydrophilic and hydrophobic parts within the polymer molecule.

In an embodiment, the lipid-polymer may be a polymer in which one or more saturated or unsaturated hydrocarbon groups having 11 to 25 carbon atoms as a hydrophobic part are introduced into a hydrophilic block which is a hydrophilic part.

The hydrophilic block may be one or more selected from the group consisting of polyalkylene glycol, polyvinyl alcohol, polyvinyl pyrrolidone, polyacrylamide, and derivatives thereof.

More specifically, the hydrophilic block may be one or more selected from the group consisting of monomethoxypolyethylene glycol, monoacetoxypolyethylene glycol, polyethylene glycol, copolymer of polyethylene and propylene glycol, and polyvinylpyrrolidone.

In an embodiment, the number average molecular weight (g/mol) of the hydrophilic block may be 200 or more, 500 or more, 1,000 or more, or 2,000 or more, and it also may be 50,000 or less, 20,000 or less, 10,000 or less, or 5,000 or less, but it is not limited thereto.

Also, if necessary, the end of the hydrophilic block may be chemically combined with a functional group or ligand capable of reaching specific tissue or cell, or a functional group capable of promoting intracellular delivery, in order to control *in vivo* distribution of the nanoparticle carrier or to increase the efficiency of delivering the nanoparticle carrier into cell. The functional group or ligand may be one or more selected from the group consisting of monosaccharides, polysaccharides, vitamins, peptides, proteins, and antibodies to cell surface receptors. More specifically, the functional group or ligand may be one or more selected from the group consisting of anisamide, vitamin B9 (folic acid), vitamin B12, vitamin A, galactose, lactose, mannose, hyaluronic acid, RGD peptide, NGR peptide, transferrin, antibody to transferrin receptor, etc.

In an embodiment, the saturated or unsaturated hydrocarbon group having 11 to 25 carbon atoms, the hydrophobic part introduced into a hydrophilic block which is the hydrophilic part, may independently be selected from the group consisting of myristoyl, dimyristoyl, lauryl, myristyl, palmityl, stearyl, arachidyl, behenyl, lignoceryl, cerotyl, myristoleyl, palmitoleyl, sapienyl, oleyl, linoleyl, arachidonyl, eicosapentaenyl, erucyl, and docosahexaenyl.

Also, in an embodiment, in the lipid-polymer, the amount ratio of the hydrophilic part and the hydrophobic part may be 10 to 90 wt%, more specifically 20 to 80 wt%, even more specifically 30 to 80 wt%, and even more specifically 40 to 80 wt% of the hydrophilic part, based on the weight of the polymer. If the ratio of the hydrophilic part is too small, the solubility of the polymer in water is low, making it difficult to form nanoparticles, and to the contrary, if it is too large, the hydrophilicity becomes too high, which may lower the stability of the nanoparticles.

In an embodiment of the present invention, the lipid-polymer may be a polyalkylene glycol (e.g., polyethylene glycol) into which saturated or unsaturated hydrocarbon group having 11 to 25 carbon atoms (e.g., myristyl group) has been introduced. For example, the lipid-polymer may be a PEG lipid (PEGylated lipid). The PEG lipid refers to a polyethylene glycol (PEG)-modified lipid, and is a type of PEG derivative having a lipid moiety attached thereto, such as DMG (dimyristoylglycerol) or DSPE (distearoyl glycerophosphoethanolamine). The PEG lipid may be used to improve the circulation time of active ingredient encapsulated in a lipid nanoparticle and to reduce non-specific uptake. The PEG lipid may be one or more selected from the group consisting of PEG-modified phosphatidylethanolamines, PEG-modified phosphatidic acids, PEG-modified ceramides, PEG-modified dialkylamines, PEG-modified diacylglycerols, and PEG-modified dialkylglycerols, and combinations thereof. More specifically, the PEG lipid may include 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-distearyl glycerol (PEG-DSG), PEG-dipalmitoyl, PEG-dioleyl, PEG-distearyl, PEG-diacylglycamide (PEG-DAG), PEG-dipalmitoyl phosphatidylethanolamine (PEG-DPPE), or PEG-1,2-dimyristyloxypropyl-3-amine (PEG-c-DMA).

The amphiphilic block copolymer may be an A-B type block copolymer comprising a hydrophilic A block and a hydrophobic B block. In an aqueous environment, the A-B type block copolymer forms core-shell type polymer nanoparticle wherein the hydrophobic B block forms the core (inner wall) and the hydrophilic A block forms the shell (outer wall).

In an embodiment, the hydrophilic A block may be one or more selected from the group consisting of polyalkylene glycol, polyvinyl alcohol, polyvinyl pyrrolidone, polyacrylamide, and derivatives thereof.

More specifically, the hydrophilic A block may be one or more selected from the group consisting of monomethoxypolyethylene glycol (mPEG), monoacetoxypolyethylene glycol, polyethylene glycol, copolymer of polyethylene and propylene glycol, and polyvinylpyrrolidone.

In an embodiment, the number average molecular weight (g/mol) of the hydrophilic A block may be 200 or more, 500 or more, 1,000 or more, or 2,000 or more, and it also may be 50,000 or less, 20,000 or less, 10,000 or less, or 5,000 or less, but it is not limited thereto.

Also, if necessary, the end of the hydrophilic A block may be chemically combined with a functional group or ligand capable of reaching specific tissue or cell, or a functional group capable of promoting intracellular delivery, in order to control *in vivo* distribution of polymer nanoparticle carrier formed by the amphiphilic block copolymer and salt of polylactic acid or to increase the efficiency of delivering the nanoparticle carrier into cell. In an embodiment, the functional group or ligand may be one or more selected from the group consisting of monosaccharides, polysaccharides, vitamins, peptides, proteins, and antibodies to cell surface receptors. More specifically, the functional group or ligand may be one or more selected from the group consisting of anisamide, vitamin B9 (folic acid), vitamin B12, vitamin A, galactose, lactose, mannose, hyaluronic acid, RGD peptide, NGR peptide, transferrin, antibody to transferrin receptor, etc.

The hydrophobic B block is a biocompatible, biodegradable polymer, and in an embodiment, it may be one or more selected from the group consisting of polyester, polyanhydride, polyamino acid, polyorthoester and polyphosphazine.

More specifically, the hydrophobic B block may be one or more selected from the group consisting of polylactide (PLA), polyglycolide, polycaprolactone, polydioxan-2-one, copolymer of polylactide and polyglycolide, copolymer of polylactide and polydioxan-2-one, copolymer of polylactide and and polycaprolactone, and a copolymer of polyglycolide and polycaprolactone.

In an embodiment, the number average molecular weight (g/mol) of the hydrophobic B block may be 200 or more, 500 or more, 1,000 or more, or 1,700 or more, and it also may be 50,000 or less, 20,000 or less, 10,000 or less, or 6,000 or less, but it is not limited thereto.

For example, the number average molecular weight combination of the hydrophilic A block-hydrophobic B block may be 2,000-6,000, 2,000-4,000, 2,000-3,000, 2,000-1,700, 2,000-1,300, etc., but it is not limited thereto.

Also, in an embodiment, in order to increase the hydrophobicity of and thereby improve the stability of the nanoparticle, the hydrophobic B block may be modified by chemically combining the hydroxyl group at the end of the hydrophobic B block with tocopherol, cholesterol, or fatty acids having 10 to 24 carbons.

In an embodiment, in the amphiphilic block copolymer, the amount ratio of the hydrophilic block (A) and the hydrophobic block (B) may be such as 20 to 70 wt% and more specifically 30 to 60 wt% of the hydrophilic block (A), based on the weight of the copolymer. If the ratio of the hydrophilic block (A) is less than 20 wt%, the solubility of the polymer in water is low, making it difficult to form nanoparticles. Thus, in order for the copolymer to have sufficient water solubility to form nanoparticles, it is preferable that the ratio of the hydrophilic block (A) be 20 wt% or more. To the contrary, if the ratio of the hydrophilic block (A) is greater than 70 wt%, the hydrophilicity becomes too high, lowering the stability of the nanoparticles and making it difficult to use as a solubilizing composition of effective ingredient/lipid complex. Thus, considering the stability of the nanoparticles, it is preferable that the ratio of the hydrophilic block (A) be 70 wt% or less.

In an embodiment, the amphiphilic block copolymer may be a biocompatible, biodegradable polymer comprising the hydrophilic block described above; and a hydrophobic block having a repeating unit represented by the following Formula 2: 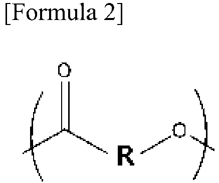 wherein, in the above Formula 2,
R represents a branched alkylene group having 3 or more carbon atoms.

In an embodiment, the number of repeating units (degree of polymerization) of the hydrophobic block having a repeating unit represented by the above Formula 2 may be, for example, 1 or more, 2 or more, 3 or more, 4 or more, 5 or more, 6 or more, 7 or more, 8 or more, 9 or more, or 10 or more, and may also be 25 or less, 24 or less, 23 or less, 22 or less, 21 or less, or 20 or less, but is not limited thereto.

In an embodiment, the number of carbon atoms of R in the above Formula 2 may be, for example, 3 or more, 4 or more, 5 or more, 6 or more, or 7 or more, and may also be 20 or less, 19 or less, 18 or less, 17 or less, 16 or less, 15 or less, 14 or less, or 13 or less, but is not limited thereto.

In an embodiment, R in the above Formula 2 may represent a branched alkylene group having 3 to 20 carbon atoms, more specifically a branched alkylene group having 3 to 17 carbon atoms, still more specifically a branched alkylene group having 3 to 15 carbon atoms, and even more specifically a branched alkylene group having 3 to 13 carbon atoms, but is not limited thereto.

In an embodiment, the hydrophobic block having a repeating unit represented by the above Formula 2 may be a biocompatible, biodegradable polymer having repeating units of a structure selected from the following, but is not limited thereto:

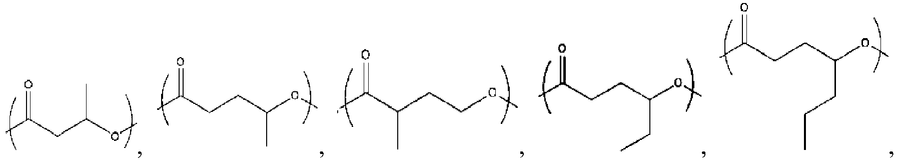

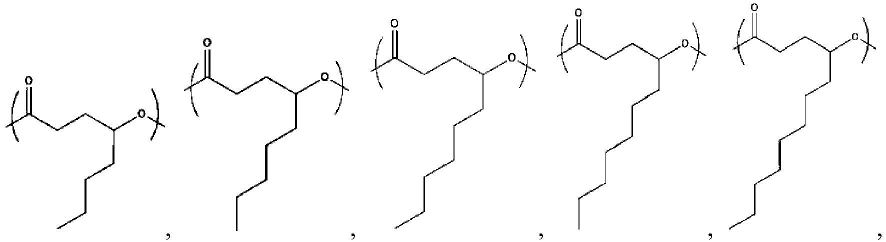

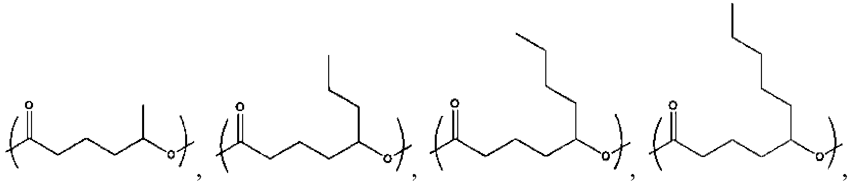

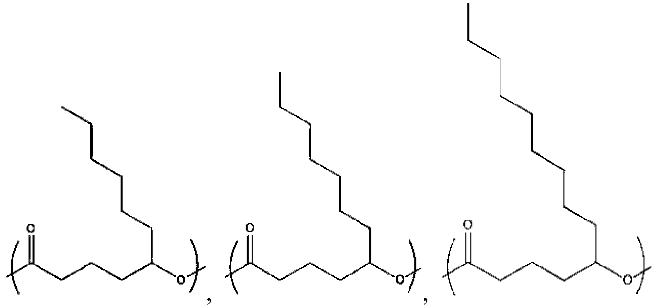

In an embodiment, the repeating unit represented by the above Formula 2 may be obtained by ring-opening polymerization of a lactone compound.

In an embodiment, the number average molecular weight (g/mol) of the hydrophobic block having a repeating unit represented by the above Formula 2 may be 80 or more, 100 or more, 150 or more, 200 or more, 500 or more, 1,000 or more, or 1,700 or more, and it also may be 50,000 or less, 20,000 or less, 10,000 or less, or 6,000 or less, but it is not limited thereto.

For example, the number average molecular weight combination of the hydrophilic block and the hydrophobic block having a repeating unit represented by the above Formula 2 may be 2,000-6,000, 2,000-4,000, 2,000-3,000, 2,000-1,700, 2,000-1,000, 2,000-800, 2,000-500, etc., but it is not limited thereto.

Also, in an embodiment, in order to increase the hydrophobicity of the hydrophobic block having a repeating unit represented by the above Formula 2 and thereby improve the stability of the nanoparticle, the hydrophobic block may be modified by chemically combining the hydroxyl group at the end of the hydrophobic block with tocopherol, cholesterol, or a fatty acid having 10 to 24 carbon atoms.

In an embodiment, in the amphiphilic block copolymer comprising the hydrophobic block having a repeating unit represented by the above Formula 2, the amount ratio of the hydrophilic block and the hydrophobic block may be such that the hydrophilic block is present in an amount of 25 to 95 wt%, specifically 40 to 90 wt%, and more specifically 50 to 80 wt%, based on the total weight of the copolymer. If the ratio of the hydrophilic block is less than 25 wt% based on the total weight of the copolymer, the solubility of the polymer in water is low, making it difficult to form nanoparticles. Thus, in order for the copolymer to have sufficient water solubility to form nanoparticles, it is preferable that the ratio of the hydrophilic block be 25 wt% or more. To the contrary, if the ratio of the hydrophilic block is greater than 95 wt% based on the total weight of the copolymer, the hydrophilicity becomes too high, lowering the stability of the polymer nanoparticles and making it difficult to use the copolymer as a solubilizing composition of effective ingredient-containing complex. Thus, considering the stability of the nanoparticles, it is preferable that the ratio of the hydrophilic block be 95 wt% or less.

In an embodiment, the amount of the polymer, which is lipid-polymer, amphiphilic block copolymer, or a combination thereof, in the composition for drug delivery of the present invention may be, based on the dry weight of the total composition, 1 wt% or more, 3 wt% or more, 5 wt% or more, 7 wt% or more, or 9 wt% or more, and it may also be 95 wt% or less, 93 wt% or less, 90 wt% or less, 88 wt% or less, or 85 wt% or less. If the amount of the polymer is too less, the size of the nanoparticle becomes too large, and thus the nanoparticle stability may be lowered and the rate of loss during filter sterilization may increase. To the contrary, if the amount of the polymer is too much, there is a concern that the amount of the effective ingredient that can be incorporated will become too less.

In the composition for drug delivery of the present invention, the effective ingredient is maintained in a state of being encapsulated within a nanoparticle structure formed by the polymer, which is lipid-polymer, amphiphilic block copolymer, or a combination thereof, and the lipid of the above Formula 1, thereby improving stability in blood or body fluids.

In an embodiment, the particle size of the nanoparticle can be defined by Z-average value, and for example, it may be 800 nm or less, 600 nm or less, 500 nm or less, 400 nm or less, 300 nm or less, 200 nm or less, or 150 nm or less, and also may be 10 nm or more, 50 nm or more, or 100 nm or more. In an embodiment, the particle size of the nanoparticle defined by Z-average value may be, for example, 10 to 800 nm, 20 to 600 nm, 30 to 500 nm, 50 to 400 nm, or 80 to 300 nm.

In an embodiment, the relative amount of the polymer, which is lipid-polymer, amphiphilic block copolymer, or a combination thereof, to the lipid of the above Formula 1 may be, based on 1 part by weight of the lipid of Formula 1, 0.01 part by weight or more, 0.02 part by weight or more, 0.03 part by weight or more, 0.04 part by weight or more, or 0.05 part by weight or more, and it may also be 50 parts by weight or less, 49 parts by weight or less, 47 parts by weight or less, 45 parts by weight or less, 43 parts by weight or less, 41 parts by weight or less, 40 parts by weight or less, 39 parts by weight or less, or 37 parts by weight or less, but it is not limited thereto.

### Optional additive component

In an embodiment, in order to increase the efficiency of *in vivo* delivery of the effective ingredient, the composition for drug delivery of the present invention may further comprise fusogenic lipid.

In an embodiment, the fusogenic lipid may be one or a combination of two or more selected from the group consisting of phospholipid, cholesterol, and tocopherol.

Specifically, the phospholipid may be one or more selected from the group consisting of phosphatidylethanolamine (PE), phosphatidylcholine (PC) and phosphatidic acid. The phosphatidylethanolamine (PE), phosphatidylcholine (PC) and phosphatidic acid may be in a form combined with one or two C₁₀₋₂₄ fatty acids. The cholesterol and tocopherol include analogues, derivatives and metabolites of each of the cholesterol and tocopherol.

More specifically, the fusogenic lipid may be one or a combination of two or more selected from the group consisting of dilauroyl phosphatidylethanolamine, dimyristoyl phosphatidylethanolamine, dipalmitoyl phosphatidylethanolamine, distearoyl phosphatidylethanolamine, dioleoyl phosphatidylethanolamine, dilinoleoyl phosphatidylethanolamine, 1-palmitoyl-2-oleoyl phosphatidylethanolamine, 1,2-diphytanoyl-3-sn-phosphatidylethanolamine, dilauroyl phosphatidylcholine, dimyristoyl phosphatidylcholine, dipalmitoyl phosphatidylcholine, distearoyl phosphatidylcholine, dioleoyl phosphatidylcholine, dilinoleoyl phosphatidylcholine, 1-palmitoyl-2-oleoyl phosphatidylcholine, 1,2-diphytanoyl-3-sn-phosphatidylcholine, dilauroyl phosphatidic acid, dimyristoyl phosphatidic acid, dipalmitoyl phosphatidic acid, distearoyl phosphatidic acid, dioleoyl phosphatidic acid, dilinoleoyl phosphatidic acid, 1-palmitoyl-2-oleoyl phosphatidic acid, 1,2-diphytanoyl-3-sn-phosphatidic acid, cholesterol, and tocopherol.

Still more specifically, the fusogenic lipid may be one or a combination of two or more selected from the group consisting of dioleoyl phosphatidylethanolamine (DOPE), 1,2-dipalmitoleoyl-sn-glycero-3-phosphocholine (DPPC), distearoyl phosphatidylcholine (DSPC), 1,2-dioleoyl-sn-glycero-3-phosphocholine (DOPC), 1,2-dipalmitoleoyl-sn-glycero-3-phosphoethanolamine (DPPE), 1,2-dioleoyl-3-trimethylammonium-propane (DOTAP), 1,2-dioleoyl-3-dimethylammonium propane (DODAP), 1,2-dioleoyl-sn-glycero-3-phosphate (18PA), cholesterol, and tocopherol.

In an embodiment of the present invention, the fusogenic lipid may be distearoyl phosphatidylcholine, cholesterol, or a combination thereof.

In an embodiment, the amount of the fusogenic lipid may be, based on the dry weight of the total composition, 1 wt% or more, 2 wt% or more, 3 wt% or more, 4 wt% or more, or 5 wt% or more, and it may also be 40 wt% or less, 35 wt% or less, 30 wt% or less, 25 wt% or less, or 20 wt% or less.

In an embodiment, the relative amount of the fusogenic lipid to the lipid of the above Formula 1 may be, based on 1 part by weight of the lipid of Formula 1, 0.05 part by weight or more, 0.06 part by weight or more, 0.07 part by weight or more, 0.08 part by weight or more, 0.09 part by weight or more, or 0.1 part by weight or more, and it may also be 6 parts by weight or less, 5.5 parts by weight or less, 5 parts by weight or less, 4.5 parts by weight or less, 4 parts by weight or less, or 3.8 parts by weight or less, but it is not limited thereto.

In an embodiment, in case of using phospholipid as the fusogenic lipid, its relative amount may be, based on 1 part by weight of the lipid of Formula 1, 0.03 part by weight or more, 0.04 part by weight or more, 0.05 part by weight or more, or 0.06 part by weight or more, and it may also be 4 parts by weight or less, 3.9 parts by weight or less, 3.7 parts by weight or less, 3.5 parts by weight or less, 3.3 parts by weight or less, 3.1 parts by weight or less, 3 parts by weight or less, 2.9 parts by weight or less, or 2.7 parts by weight or less, but it is not limited thereto.

In an embodiment, in case of using cholesterol as the fusogenic lipid, its relative amount may be, based on 1 part by weight of the lipid of Formula 1, 0.02 part by weight or more, 0.03 part by weight or more, or 0.04 part by weight or more, and it may also be 2 parts by weight or less, 1.9 parts by weight or less, 1.7 parts by weight or less, 1.5 parts by weight or less, 1.3 parts by weight or less, or 1.1 parts by weight or less, but it is not limited thereto.

### Composition and preparation method thereof

The composition for drug delivery according to the present invention can be administered through routes of administration such as blood vessels, muscles, mucous membranes, subcutaneous, intradermal, oral, bone, transdermal, or local tissues, and can be formulated into various oral or parenteral formulations suitable for such routes of administration. Examples of the oral formulations include various ones such as tablets, capsules, powder formulations, liquid formulations, etc., and examples of the parenteral formulations include various ones such as eye drops, injections, etc., and in an embodiment, the composition may be an injection formulation. For example, when the composition according to the present invention is freeze-dried, it can be reconstituted with distilled water for injection, 0.9% physiological saline, 5% aqueous dextrose solution, etc. to produce an injection formulation.

The present invention also provides a method for preparing a composition for drug delivery, comprising the steps of: (a) preparing a solution in which a lipid represented by the above Formula 1; and lipid-polymer, amphiphilic block copolymer, or a mixture thereof; are dissolved in a water-miscible organic solvent; and (b) to the solution prepared in step (a), adding effective ingredient selected from nucleic acid, polypeptide, virus, or combination thereof, and mixing them.

In an embodiment, the water-miscible organic solvent in step (a) may be ethanol.

In an embodiment, step (a) can be performed in a solution under acidic condition.

In an embodiment, step (b) may comprise: (b-1) a step of preparing a buffer solution containing the effective ingredient; and (b-2) a step of adding the buffer solution of the effective ingredient prepared in step (b-1) to the solution prepared in step (a), and mixing them.

In an embodiment, the mixing ratio of the buffer solution of the effective ingredient prepared in step (b-1) to the solution prepared in step (a) may be 1:1 to 1:5 in volume ratio, and more specifically, it may be 1:2 to 1:4.

In another embodiment, step (b) may comprise: (b-1) a step of adding the effective ingredient to the solution prepared in step (a); and (b-2) a step of adding a buffer solution to the resulting mixture of step (b-1) and mixing them.

In an embodiment, the method for preparing a composition for drug delivery may further comprise a step of adding a buffer for modifying pH, water for injection, or a combination thereof to the resulting mixture of step (b).

In another embodiment, the method for preparing a composition for drug delivery may further comprise a step of removing the solvent from the resulting mixture of step (b) and then adding a freeze-drying aid thereto and freeze-drying the resulting mixture.

The freeze-drying aid is added to help the freeze-dried composition maintain a cake shape or to help the composition dissolve uniformly within a short period of time during the reconstitution process after freeze-drying, and specifically, it may be one or more selected from the group consisting of sugars, amino acids, polymers and proteins; for example, it may be one or more selected from the group consisting of lactose, mannitol, sorbitol, and sucrose. The amount of the freeze-drying aid may be 1 to 90 wt%, more specifically 10 to 60 wt%, based on the total dry weight of the freeze-dried composition.

The present invention will be explained below in more detail with reference to the following Examples. However, the Examples are only to illustrate the invention, and the scope of the present invention is not limited thereby in any manner.

### [EXAMPLES]

### Lipid Preparation Example 1

The compound of the following Formula A was prepared as follows.

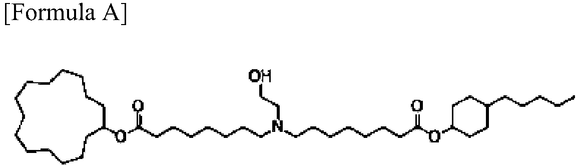

### 1-2. Synthesis of 4-pentylcyclohexyl 8-bromooctanoate

In a 250 mL 3-neck round bottom flask (RBF), 8-bromooctanoic acid (2.00 g, 8.96 mmol, 1.00 eq), dichloromethane (DCM) (40 mL), and dimethylformamide (DMF) (0.5 mL) were added together, and oxalyl chloride (2.28 g, 17.9 mmol, 2.00 eq) was added thereto at 0 °C under a nitrogen environment. The resulting mixture was stirred at 25 °C for 4 hours under a nitrogen environment, and then 4-pentylcyclohexan-1-ol (2.29 g, 13.5 mmol, 1.50 eq) and triethylamine (TEA) (1.36 g, 13.5 mmol, 1.50 eq) were added, and the mixture was stirred at 25 °C for an additional 12 hours under a nitrogen environment. The reaction mixture was concentrated in vacuo, and the concentrated residue was purified using a silica column with petroleum ether:ethyl acetate (EtOAc) = 1:0 → 50:1 to obtain 4-pentylcyclohexyl 8-bromooctanoate (2.46 g, 6.55 mmol, 73.1% yield) as a pale yellow oil.

¹H NMR (400 MHz, CHLOROFORM-*d*): *δ* 0.76 - 0.86 (m, 3 H) 0.88 - 1.94 (m, 28 H) 2.21 (dt, 2 H) 3.33 (td, 2 H) 4.52 - 4.66 (m, 1 H) 4.87 - 4.95 (m, 1 H)

### 1-3. Synthesis of 4-pentylcyclohexyl 8-((2-hydroxyethyl)amino)octanoate

In a 100 mL 3-neck RBF, 4-pentylcyclohexyl 8-bromooctanoate (2.46 g, 6.55 mmol, 1.00 eq), 2-aminoethan-1-ol (2.00 g, 32.8 mmol, 5.00 eq), and ethanol (EtOH) (50 mL) were added, and the mixture was stirred at 80 °C for 16 hours under a nitrogen environment. The reaction mixture was concentrated in vacuo, and the concentrated residue was purified using a silica column with petroleum ether:EtOAc = 1:0 → 50:1 to obtain 4-pentylcyclohexyl 8-((2-hydroxyethyl)amino)octanoate (2.00 g, 5.62 mmol, 85.8 % yield) as a yellow solid.

¹H NMR (400 MHz, CHLOROFORM-*d*): *δ* 0.76 - 0.86 (m, 3 H) 0.88 - 1.94 (m, 27 H) 2.32 (t, 2 H) 2.51 (t, 2 H) 2.71 (t, 2H), 3.54 (d, 2 H) 3.91 - 4.00 (m, 1 H) 4.11 - 4.31 (m, 1 H)

### 1-4. Synthesis of cyclopentadecyl 8-bromooctanoate

In a 250 mL 3-neck RBF, cyclopentadecanol (5.00 g, 22.1 mmol, 1.00 eq), 8-bromooctanoic acid (4.93 g, 22.1 mmol, 1 eq), sulfuric acid (H2SO4) (217 mg, 2.21 mmol, 0.10 eq), and toluene (100 mL) were added, and the mixture was stirred at 120 °C for 16 hours under a nitrogen environment. After evaporating the solvent, the residue was purified using a silica column with petroleum ether:EtOAc = 1:0 → 50:1 to obtain cyclopentadecyl 8-bromooctanoate (2.60 g, 6.03 mmol, 27.3% yield) as a colorless oil.

¹H NMR (400 MHz, CHLOROFORM-*d*): δ 4.89 (quin, 1H), 3.41 (dt, 2H), 2.28 (t, 2H), 1.85 (quin, 2H), 1.73 - 1.16 (m, 36H)

### 1-5. Synthesis of the compound of Formula A

In a 100 mL 3-neck RBF, cyclopentadecyl 8-bromooctanoate (1.60 g, 3.71 mmol, 1.00 eq), 4-pentylcyclohexyl 8-((2-hydroxyethyl)amino)octanoate (1.32 g, 3.71 mmol, 1.00 eq), N,N-diisopropylethylamine (DIEA) (527 mg, 4.08 mmol, 1.10 eq), and EtOH (30 mL) were added, and the mixture was stirred at 80 °C for 48 hours. Subsequently, after evaporating the solvent, the residue was purified using a silica column with petroleum ether:EtOAc = 10:1 → 1:1, and purified again by prep-HPLC (Folic Acid condition) and washed with NaHCO₃ aqueous solution (300 mL), and then the organic layer was concentrated and extracted with DCM (200 mL x 2). The organic layer was dried over anhydrous Na₂SO₄ and filtered, and the filtrate was concentrated to obtain the compound of Formula A (0.240 g, 340 µmol, 9.16% yield) as a yellow oil.

¹H NMR (400 MHz, CHLOROFORM-*d*): *δ* 4.91 (br s, 1H), 4.82 (quin, 1H), 3.46 (br t, 2H), 2.51 (br d, 2H), 2.37 (br t, 4H), 2.21 (td, 4H), 1.58 - 1.42 (m, 14H), 1.33 - 1.17 (m, 52H), 0.83 - 0.80 (m, 3H).

### Lipid Preparation Example 2

The compound of the following Formula B was prepared as follows.

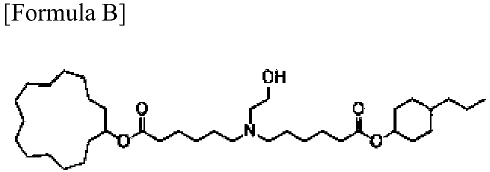

### 2-2. Synthesis of cyclopentadecyl 6-bromohexanoate

In a 100 mL 3-neck RBF, cyclopentadecanol (5.00 g, 22.1 mmol, 1.00 eq), 6-bromohexanoic acid (6.46 g, 33.1 mmol, 1.50 eq), 1-ethyl-3-(3-dimethylaminopropyl)carbodiimide (EDCI) (5.08 g, 26.5 mmol, 1.20 eq), 4-dimethylaminopyridine (DMAP) (540 mg, 4.42 mmol, 0.20 eq), triethylamine (TEA) (4.47 g, 44.2 mmol, 6.15 mL, 2.00 eq), and dichloromethane (DCM) (50 mL) were added together, and the resulting mixture was purged three times with nitrogen gas. The resulting mixture was stirred at 25 °C for 16 hours under a nitrogen atmosphere, and then water (200 mL) was added thereto at 20 °C to quench the reaction. The resulting mixture was extracted with DCM 600 mL (200 mL x 3), and the combined organic layers were dried over Na₂SO₄, filtered, and concentrated in vacuo. The concentrated residue was purified using a silica column with petroleum ether:ethyl acetate (EtOAc) = 100:1 → 1:1 to obtain cyclopentadecyl 6-bromohexanoate (2.20 g, 5.45 mmol, 24.7% yield) as a yellow oil.

¹H NMR (400 MHz, CHLOROFORM-*d*): *δ* 4.83 (quin, 1H), 3.50 - 3.30 (m, 2H), 2.23 (t, 2H), 1.87 - 1.68 (m, 2H), 1.63 - 1.37 (m, 10H), 1.26 (br s, 22H)

### 2-3. Synthesis of 4-propylcyclohexyl 6-bromohexanoate

In a 250 mL 3-neck RBF, 4-propylcyclohexan-1-ol (10.0 g, 70.3 mmol, 1.00 eq), 6-bromohexanoic acid (16.5 g, 84.4 mmol, 1.20 eq), EDCI (20.2 g, 105 mmol, 1.50 eq), DMAP (8.59 g, 70.3 mmol, 1.00 eq), and TEA (7.11 g, 70.3 mmol, 9.79 mL, 1.00 eq) were added to DCM (100 mL), and the resulting mixture was purged three times with nitrogen gas. The resulting mixture was stirred at 25 °C for 16 hours under a nitrogen atmosphere, and then water (100 mL) was added thereto at 20 °C to quench the reaction. The resulting mixture was extracted with DCM 600 mL (200 mL x 3), and the combined organic layers were concentrated in vacuo. The concentrated residue was purified using a silica column with petroleum ether:EtOAc = 100:1 → 10:1 to obtain 4-propylcyclohexyl 6-bromohexanoate (6.80 g, 21.3 mmol, 30.3% yield) as a yellow oil.

¹H NMR (400 MHz, CHLOROFORM-*d*): δ 0.76 - 0.86 (m, 3 H) 0.88 - 1.94 (m, 19 H) 2.21 (dt, 2 H) 3.33 (td, 2 H) 4.52 - 4.66 (m, 1 H)

### 2-4. Synthesis of 4-propylcyclohexyl 6-((2-hydroxyethyl)amino)hexanoate

In a 250 mL 3-neck RBF, 4-propylcyclohexyl 6-bromohexanoate (3.00 g, 9.40 mmol, 1.00 eq), 2-aminoethan-1-ol (2.87 g, 47.0 mmol, 2.84 mL, 5.00 eq), and EtOH (60 mL) were added together, and the resulting mixture was purged three times with nitrogen gas. The resulting mixture was stirred at 85 °C for 16 hours under a nitrogen atmosphere. The reaction mixture was cooled to room temperature and concentrated to remove the solvent. The resulting residue was purified by column chromatography (SiO₂, DCM:MeOH = 100:1 → 1:1) to obtain 4-propylcyclohexyl 6-((2-hydroxyethyl)amino)hexanoate (0.70 g, 2.34 mmol, 24.9% yield) as a yellow oil.

¹H NMR (400 MHz, CHLOROFORM-*d*): δ 5.04 - 4.60 (m, 1H), 3.70 - 3.61 (m, 2H), 2.79 (t, 2H), 2.64 (dt, 2H), 2.35 - 2.24 (m, 2H), 2.01 - 1.94 (m, 2H), 1.87 - 1.74 (m, 2H), 1.65 (qd, 2H), 1.56 - 1.48 (m, 4H), 1.41 - 1.16 (m, 9H), 1.07 - 0.94 (m, 1H), 0.89 (dt, 3H)

### 2-5. Synthesis of the compound of Formula B

In a 50 mL 3-neck RBF, 4-propylcyclohexyl 6-((2-hydroxyethyl)amino)hexanoate (371 mg, 1.24 mmol, 1.00 eq), 4-propylcyclohexyl 6-bromohexanoate (0.50 g, 1.24 mmol, 1.00 eq), and DIEA (800 mg, 6.20 mmol, 1.08 mL, 5.00 eq) were added together with 1,4-dioxane (10 mL), and the resulting mixture was purged three times with nitrogen gas. The resulting mixture was stirred at 95 °C for 16 hours under a nitrogen atmosphere. The reaction mixture was cooled to room temperature and concentrated under reduced pressure to remove the solvent. The resulting residue was purified by column chromatography (SiO₂, DCM:MeOH = 100:1 → 10:1) to obtain the compound of Formula B (0.21 g, 337.64 µmol, 27.2% yield) as a yellow oil.

¹H NMR (400 MHz, CHLOROFORM-*d*): δ 4.94 - 4.52 (m, 2H), 3.79 (br s, 2H), 3.06 - 2.63 (m, 6H), 2.29 - 2.17 (m, 4H), 1.93 - 1.81 (m, 1H), 1.79 - 1.37 (m, 17H), 1.36 - 1.09 (m, 34H), 0.99 - 0.86 (m, 1H), 0.85 - 0.76 (m, 3H)

### Lipid Preparation Example 3

The compound of the following Formula C was prepared as follows.

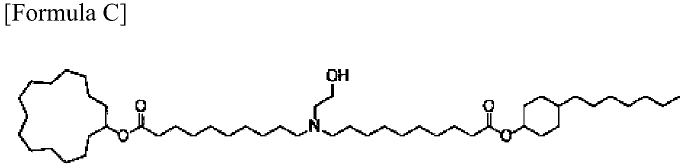

### 3-2. Synthesis of cyclopentadecyl 10-bromodecanoate

In a 100 mL 3-neck RBF, cyclopentadecanol (5.00 g, 22.1 mmol, 1.00 eq), 10-bromodecanoic acid (8.32 g, 33.1 mmol, 1.50 eq), DMAP (540 mg, 4.42 mmol, 0.20 eq), EDCI (5.08 g, 26.5 mmol, 1.20 eq), and TEA (4.47 g, 44.2 mmol, 2.00 eq) were added together with DCM (50 mL), and the resulting mixture was purged three times with nitrogen gas. The resulting mixture was stirred at 50 °C for 16 hours under a nitrogen atmosphere. The reaction mixture was cooled to 20 °C, and then water was added thereto to quench the reaction. The resulting mixture was extracted with DCM 600 mL (200 mL × 3), and the combined organic layers were dried over Na₂SO₄ and filtered. The filtrate was concentrated, and the resulting residue was purified by column chromatography (SiO₂, petroleum ether:EtOAc = 100:1 → 1:1) to obtain cyclopentadecyl 10-bromodecanoate (2.60 g, 5.66 mmol, 25.6% yield) as a yellow oil.

¹H NMR (400 MHz, CHLOROFORM-*d*): *δ* 4.90 (quin, 1H), 3.58 - 3.36 (m, 2H), 2.27 (t, 2H), 1.90 - 1.72 (m, 2H), 1.64 - 1.53 (m, 6H), 1.43 - 1.28 (m, 34H)

### 3-3. Synthesis of 4-heptylcyclohexan-1-ol

In a 500 mL 3-neck RBF, LiAlH₄ (2.50 M in THF, 61.1 mL, 1.20 eq) was added together with THF (250 mL), and the resulting mixture was cooled to 0 °C. A solution of 4-heptylcyclohexan-1-one (25.0 g, 127 mmol, 1.00 eq) in THF (250 mL) was slowly added thereto over 20 minutes under a nitrogen atmosphere. The resulting mixture was then warmed to 25 °C and stirred for 3 hours under a nitrogen atmosphere. The reaction mixture was cooled to 0 °C, and water (120 mL) was added thereto under a nitrogen atmosphere while carefully controlling excessive foaming. Thereafter, 15% aq. NaOH (12 mL) was slowly added to the reaction mixture at 0 °C, followed by the addition of water (36 mL) after 5 minutes at the same temperature. The resulting mixture was then slowly warmed to 25 °C and stirred for 15 minutes. The resulting mixture was filtered, and the filter cake was concentrated in vacuo to obtain 4-heptylcyclohexan-1-ol (50.0 g, 252 mmol, 98.9% yield) as a colorless powder.

¹H NMR (400 MHz, CHLOROFORM-*d*): *δ* 3.47 (tt, H), 1.95 - 1.84 (m, 2H), 1.75 - 1.65 (m, 2H), 1.42 (s, 1H), 1.24 - 1.14 (m, 14H), 1.11 - 1.08 (m, 2H), 0.83 - 0.80 (m, 3H)

### 3-4. Synthesis of 4-heptylcyclohexyl 10-bromodecanoate

In a 250 mL 3-neck RBF, 4-heptylcyclohexan-1-ol (10.0 g, 50.4 mmol, 1.00 eq), 10-bromodecanoic acid (15.2 g, 60.5 mmol, 1.20 eq), EDCI (14.5 g, 75.6 mmol, 1.50 eq), DMAP (6.16 g, 50.4 mmol, 1.00 eq), and TEA (5.10 g, 50.4 mmol, 1.00 eq) were added together with DCM (100 mL), and the resulting mixture was purged three times with nitrogen gas. The resulting mixture was stirred at 25 °C for 16 hours under a nitrogen atmosphere. The reaction mixture was neutralized by the addition of water (200 mL) at 20 °C, and the resulting mixture was extracted with DCM 600 mL (200 mL x 3). The combined organic layers were concentrated in vacuo, and the concentrated residue was purified by column chromatography (SiO₂, petroleum ether:EtOAc = 100:1 → 1:1) to obtain 4-heptylcyclohexyl 10-bromodecanoate (6.60 g, 15.3 mmol, 30.3% yield) as a white solid.

¹H NMR (400 MHz, CHLOROFORM-*d*): *δ* 4.59 (tt, 1H), 3.46 (t, 1H), 3.33 (t, 1H), 2.19 (t, 2H), 1.87 (br dd, 2H), 1.82 - 1.67 (m, 4H), 1.58 - 1.50 (m, 2H), 1.38 - 1.32 (m, 2H), 1.21 (br d, 23H), 0.97 - 0.86 (m, 2H), 0.81 (t, 3H)

### 3-5. Synthesis of 4-heptylcyclohexyl 10-((2-hydroxyethyl)amino)decanoate

In a 250 mL 3-neck RBF, 4-heptylcyclohexyl 10-bromodecanoate (2.30 g, 5.33 mmol, 1.00 eq) and 2-aminoethan-1-ol (1.63 g, 26.6 mmol, 5.00 eq) were added together with EtOH (60 mL), and the resulting mixture was purged three times with nitrogen gas. The resulting mixture was stirred at 95 °C for 16 hours under a nitrogen atmosphere. The reaction mixture was concentrated in vacuo, and the concentrated residue was purified by column chromatography (SiO₂, DCM:MeOH = 100:1 → 1:1) to obtain 4-heptylcyclohexyl 10-((2-hydroxyethyl)amino)decanoate (1.30 g, 3.16 mmol, 59.2% yield) as a white solid.

¹H NMR (400 MHz, CHLOROFORM-*d*): *δ* 4.66 (tt, 1H), 3.83 - 3.73 (m, 2H), 2.96 - 2.88 (m, 2H), 2.81 - 2.73 (m, 2H), 2.26 (t, 2H), 1.95 (br dd, 2H), 1.78 (br d, 2H), 1.62 (td, 4H), 1.28 (br d, 24H), 1.18 (br d, 2H), 1.04 - 0.96 (m, 2H), 0.89 (t, 3H)

### 3-6. Synthesis of the compound of Formula C

In a 50 mL 3-neck RBF, 4-heptylcyclohexyl 10-((2-hydroxyethyl)amino)decanoate (0.80 g, 1.94 mmol, 1.00 eq), cyclopentadecyl 10-bromodecanoate (893 mg, 1.94 mmol, 1.00 eq), and DIEA (1.26 g, 9.72 mmol, 1.69 mL, 5.00 eq) were added together with EtOH (3 mL), and the resulting mixture was purged three times with nitrogen gas. The resulting mixture was stirred at 95 °C for 16 hours under a nitrogen atmosphere. The reaction mixture was cooled to room temperature and concentrated in vacuo. The concentrated residue was purified by chromatography (SiO₂, DCM:MeOH = 100:1 → 10:1) to obtain the compound of Formula C (0.35 g, 447 µmol, 22.9% yield) as a white solid.

¹H NMR (400 MHz, CHLOROFORM-*d*): *δ* 4.96 - 4.61 (m, 2H), 3.77 - 3.52 (m, 2H), 2.84 - 2.37 (m, 6H), 2.27 (dt, 4H), 2.01 - 1.92 (m, 2H), 1.79 (br d, 2H), 1.68 - 1.46 (m, 18H), 1.37 - 1.25 (m, 56H), 1.00 (br d, 1H), 0.89 (br t, 3H)

### Lipid Preparation Example 4

The compound of the following Formula D was prepared as follows.

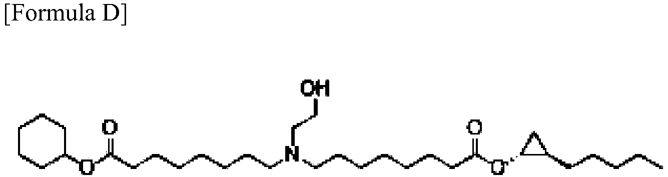

### 4-2. Synthesis of (E)-2-(hept-1-en-1-yl)-4,4,5,5-tetramethyl-1,3,2-dioxaborolane

In a 500 mL 3-neck RBF, hept-1-yne (64.4 g, 670 mmol, 1.00 eq), TEA (Et₃N, 6.78 g, 67.0 mmol, 0.10 eq), 4,4,5,5-tetramethyl-1,3,2-dioxaborolane (90.0 g, 703 mmol, 1.05 eq), and chlorozirconocene cyclopentane complex (17.9 g, 67.0 mmol, 0.10 eq) were added together, and the resulting mixture was purged three times with nitrogen gas. The resulting mixture was stirred at 60 °C for 16 hours under a nitrogen atmosphere. The reaction mixture was cooled to room temperature and filtered. The filtrate was concentrated in vacuo, and the concentrated residue was purified by silica column chromatography (petroleum ether:EtOAc = 10:1) to obtain (E)-2-(hept-1-en-1-yl)-4,4,5,5-tetramethyl-1,3,2-dioxaborolane (102 g, 455 mmol, 67.9% yield) as a yellow oil.

¹H NMR: (400 MHz, CHLOROFORM-*d*): δ 6.64 (td, 1H), 5.43 (td, 1H), 2.19 - 2.12 (m, 2H), 1.44 - 1.39 (m, 2H), 1.30 (br d, 4H), 1.27 (s, 12H), 0.90 - 0.87 (m, 3H)

### 4-3. Synthesis of 4,4,5,5-tetramethyl-2-((1R,2R)-2-pentylcyclopropyl)-1,3,2-dioxaborolane

In a 1000 mL 3-neck RBF, diethyl zinc (1 M, 178.5 mL, 2.00 eq) was added together with DCM (80 mL) distilled under a nitrogen atmosphere. A solution of trifluoroacetic acid (TFA) (20.4 g, 178 mmol, 2.00 eq) in DCM (40 mL) was added thereto portionwise at 0 °C. In addition, a solution of diiodomethane (47.8 g, 178 mmol, 2.00 eq) in DCM (40 mL) was added thereto while stirring at 0 °C over 30 minutes. The resulting mixture was stirred for an additional 30 minutes, and then a solution of (E)-2-(hept-1-en-1-yl)-4,4,5,5-tetramethyl-1,3,2-dioxaborolane (20.0 g, 89.2 mmol, 1.00 eq) in DCM (40 mL) was added thereto at 0 °C. The resulting mixture was stirred at 25 °C for 2 hours under a nitrogen atmosphere. The reaction mixture was quenched with water and extracted with DCM 3000 mL (1000 mL x 3). The combined organic layers were dried over Na₂SO₄ and filtered. The filtrate was concentrated in vacuo, and the concentrated residue was purified by silica column chromatography (petroleum ether:EtOAc = 10:1 → 1:100) to obtain 4,4,5,5-tetramethyl-2-((1R,2R)-2-pentylcyclopropyl)-1,3,2-dioxaborolane (14.0 g, 58.8 mmol, 65.9% yield) as a yellow oil.

¹H NMR (400 MHz, CHLOROFORM-*d*): δ 1.42 - 1.37 (m, 2H), 1.32 - 1.26 (m, 6H), 1.26 - 1.24 (m, 2H), 1.22 (s, 10H), 0.89 (br t, 4H), 0.67 (dt, 1H), 0.43 - 0.33 (m, 1H), -0.42 (td, 1H)

### 4-4. Synthesis of (1R,2R)-2-pentylcyclopropan-1-ol

In a 500 mL 3-neck RBF, 4,4,5,5-tetramethyl-2-((1R,2R)-2-pentylcyclopropyl)-1,3,2-dioxaborolane (8.00 g, 33.6 mmol, 1.00 eq) and THF (160 mL) were added together, and NaOH (2.69 g, 67.2 mmol, 2.00 eq) was slowly added thereto at 0 °C. H₂O₂ (7.87 g, 69.4 mmol, 30% purity, 2.07 eq) was then added thereto at 0 °C, and the resulting mixture was stirred at 25 °C for 16 hours. Thereafter, ice water containing Na₂SO₃ (2.00 eq) was added to the reaction mixture to quench the reaction, and the resulting mixture was extracted with DCM 90 mL (30 mL x 3). The combined organic layers were dried over Na₂SO₄ and filtered. The filtrate was concentrated in vacuo, and the concentrated residue was purified by silica column chromatography (petroleum ether:EtOAc = 10:1 → 1:100) to obtain (1R,2R)-2-pentylcyclopropan-1-ol (2.50 g, 19.5 mmol, 58.1% yield) as a yellow oil.

¹H NMR (400 MHz, CHLOROFORM-*d*): *δ* 3.20 (td, 1H), 1.84 - 1.67 (m, 1H), 1.41 - 1.35 (m, 2H), 1.33 - 1.26 (m, 4H), 1.23 - 1.06 (m, 2H), 0.94 - 0.87 (m, 4H), 0.68 (ddd, 1H), 0.31 (q, 1H)

### 4-5. Synthesis of (1R,2R)-2-pentylcyclopropyl 8-bromooctanoate

In a 100 mL 3-neck RBF, (1R,2R)-2-pentylcyclopropan-1-ol (2.50 g, 19.5 mmol, 1.00 eq) was added together with DCM (25 mL), and 8-bromooctanoic acid (5.22 g, 23.4 mmol, 1.20 eq), EDCI (4.49 g, 23.4 mmol, 1.20 eq), DMAP (476 mg, 3.90 mmol, 0.20 eq), and Et₃N (3.95 g, 39.0 mmol, 2.00 eq) were added thereto. The resulting mixture was purged three times with nitrogen gas and stirred at 25 °C for 16 hours under a nitrogen atmosphere. The reaction mixture was filtered, and the filtrate was concentrated in vacuo. The concentrated residue was purified by silica column chromatography (petroleum ether:EtOAc = 10:1 → 1:100) to obtain (1R,2R)-2-pentylcyclopropyl 8-bromooctanoate (3.50 g, 10.5 mmol, 53.9% yield) as a yellow oil.

¹H NMR (400 MHz, CHLOROFORM-*d*): δ 3.85 - 3.79 (m, 1H), 3.41 (t, 2H), 2.26 (t, 2H), 1.86 (quin, 2H), 1.65 - 1.58 (m, 2H), 1.46 - 1.38 (m, 4H), 1.36 - 1.27 (m, 10H), 1.00 (ddd, 1H), 0.89 (br t, 3H), 0.78 (ddd, 1H), 0.52 (q, 1H)

### 4-6. Synthesis of cyclohexyl 8-bromooctanoate

In a 1000 mL 3-neck RBF, 8-bromooctanoic acid (15.0 g, 67.2 mmol, 1.00 eq) was added together with DCM (150 mL), and trifluoroacetic anhydride (TFAA) (14.1 g, 67.2 mmol, 1.00 eq) was added thereto under a nitrogen atmosphere. The resulting mixture was stirred at 0-25 °C for 2.5 hours under a nitrogen atmosphere. Cyclohexanol (33.7 g, 336 mmol, 35.1 mL, 5.00 eq) was then added thereto at 0 °C, and the resulting mixture was stirred at 25 °C for 16 hours under a nitrogen atmosphere. The reaction mixture was neutralized with water (100 mL) at 20 °C, and the resulting mixture was extracted with DCM 600 mL (200 mL x 3). The combined organic layers were dried over Na₂SO₄ and filtered. The filtrate was concentrated in vacuo, and the concentrated residue was purified by silica column chromatography (SiO₂, petroleum ether:EtOAc = 100:1 → 10:1) to obtain cyclohexyl 8-bromooctanoate (7.10 g, 23.3 mmol, 34.6% yield) as a colorless oil.

¹H NMR (400 MHz, CHLOROFORM-*d*): *δ* 4.77 (br d, 1H), 3.42 (t, 2H), 2.30 (t, 2H), 1.93 - 1.82 (m, 4H), 1.74 (br dd, 2H), 1.67 - 1.55 (m, 4H), 1.47 - 1.31 (m, 10H)

### 4-7. Synthesis of cyclohexyl 8-((2-hydroxyethyl)amino)octanoate

In a 250 mL 3-neck RBF, cyclohexyl 8-bromooctanoate (3.00 g, 9.83 mmol, 1.00 eq), 2-aminoethan-1-ol (3.00 g, 49.1 mmol, 5.00 eq), and Na₂CO₃ (1.04 g, 9.83 mmol, 1.00 eq) were added together with 1,4-dioxane (60 mL), and the resulting mixture was purged three times with nitrogen gas. The resulting mixture was stirred at 80 °C for 16 hours under a nitrogen atmosphere. The reaction mixture was filtered, and the filtrate was concentrated in vacuo. The concentrated residue was purified by silica column chromatography (DCM:MeOH = 100:1 → 10:1) to obtain cyclohexyl 8-((2-hydroxyethyl)amino)octanoate (2.80 g, 9.81 mmol, 99.8% yield) as a yellow oil.

¹H NMR (400 MHz, CHLOROFORM-*d*): *δ* 4.77 (td, 1H), 3.75 - 3.67 (m, 2H), 2.91 - 2.80 (m, 5H), 2.68 (t, 2H), 2.29 (t, 2H), 1.88 - 1.82 (m, 2H), 1.77 - 1.70 (m, 2H), 1.66 - 1.60 (m, 2H), 1.55(br d, 2H), 1.46 - 1.28 (m, 12H)

### 4-8. Synthesis of the compound of Formula D

In a 50 mL 3-neck RBF, cyclohexyl 8-((2-hydroxyethyl)amino)octanoate (942 mg, 3.30 mmol, 1.10 eq) was added together with EtOH (10 mL), and (1R,2R)-2-pentylcyclopropyl 8-bromooctanoate (1.00 g, 3.00 mmol, 1.00 eq) and DIEA (1.94 g, 15.00 mmol, 5.00 eq) were added thereto. The resulting mixture was stirred at 90 °C for 16 hours under a nitrogen atmosphere. The reaction mixture was filtered, and the filtrate was concentrated in vacuo. The concentrated residue was purified by silica column chromatography (DCM:MeOH = 10:1 → 1:100) to obtain the compound of Formula D (0.15 g, 279 µmol, 9.30% yield) as a yellow oil.

¹H NMR (400 MHz, CHLOROFORM-*d*): *δ* 5.23 - 4.94 (m, 2H), 4.63 - 4.56 (m, 1H), 4.12 (br s, 2H), 3.68 - 3.57 (m, 2H), 3.56 - 3.37 (m, 4H), 2.40 - 2.27 (m, 2H), 2.04 (br d, 6H), 1.91 - 1.76 (m, 2H), 1.74 - 1.55 (m, 17H), 1.54 - 1.46 (m, 2H), 1.43 - 1.25 (m, 5H), 1.21 (s, 2H), 0.90 (d, 6H)

### Lipid Preparation Example 5

The compound of the following Formula E was prepared as follows.

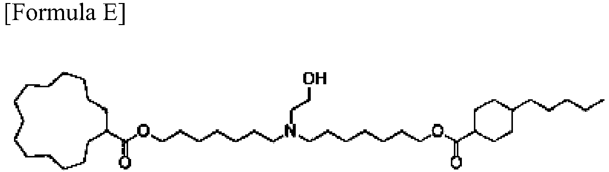

### 5-2. Synthesis of cyclopentadecanecarbonitrile

In a 2000 mL 3-neck RBF, cyclopentadecanone (25.0 g, 111 mmol, 1.00 eq), potassium 2-methylpropan-2-olate (25.0 g, 223 mmol, 2.00 eq), and 2-methylpropan-2-ol (250 mL) were added together with THF (500 mL), and the resulting mixture was cooled to 0 °C. 1-(Isocyanomethylsulfonyl)-4-methylbenzene (32.6 g, 167 mmol, 1.50 eq) was slowly added thereto over 1 hour at 0 °C, and the resulting mixture was stirred at 20 °C for 11 hours. The reaction mixture was diluted with H₂O (400 mL) and extracted with EtOAc (400 mL). The organic layer was washed with H₂O (400 mL), dried over Na₂SO₄, and filtered. The filtrate was concentrated in vacuo, and the concentrated residue was purified by silica column chromatography (petroleum ether:EtOAc = 1:0 → 5:1) to obtain cyclopentadecanecarbonitrile (20 g, 84.96 mmol, 76.25% yield) as a colorless oil.

¹H NMR (400 MHz, CHLOROFORM-*d*): *δ* 1.33 (br s, 20 H) 1.44 - 1.53 (m, 4 H) 1.67 (q, 4 H) 2.59 (quin, 1 H)

### 5-3. Synthesis of cyclopentadecanecarboxylic acid

In a 250 mL 3-neck RBF, cyclopentadecanecarbonitrile (14.0 g, 59.5 mmol, 1.00 eq), KOH (6.00 M, 69.4 mL, 7.00 eq), and EtOH (70 mL) were added together, and the resulting mixture was purged three times with nitrogen gas. The resulting mixture was stirred at 100 °C for 16 hours under a nitrogen atmosphere. The reaction mixture was concentrated in vacuo, and the concentrated residue was acidified to pH 4 with 4 M aqueous hydrochloric acid (50 mL). The resulting mixture was extracted with EtOAc 150 mL (50 mL x 3). The combined organic layers were dried over Na₂SO₄ and filtered. The filtrate was concentrated in vacuo, and the concentrated residue was purified by silica column chromatography (petroleum ether:EtOAc = 10:1 → 1:1) to obtain cyclopentadecanecarboxylic acid (1.20 g, 4.72 mmol, 7.93% yield) as a white solid.

¹H NMR (400 MHz, CHLOROFORM-*d*): *δ* ppm 1.27 - 1.46 (m, 24 H) 1.55 - 1.72 (m, 4 H) 2.44 (quin, 1 H) 10.15 - 11.75 (m, 1 H)

### 5-4. Synthesis of 7-bromoheptyl cyclopentadecanecarboxylate

In a 50 mL 3-neck RBF, cyclopentadecanecarboxylic acid (1.20 g, 4.72 mmol, 1.10 eq) was added together with toluene (12 mL), and 7-bromoheptan-1-ol (837 mg, 4.29 mmol, 1.00 eq) was added thereto. H₂SO₄ (84.1 mg, 858 µmol, 45.7 µL, 0.20 eq) was then added thereto, and the resulting mixture was purged three times with nitrogen gas. The resulting mixture was stirred at 120 °C for 16 hours under a nitrogen atmosphere. The reaction mixture was concentrated in vacuo, and the concentrated residue was purified by silica column chromatography (petroleum ether:EtOAc = 10:1) to obtain 7-bromoheptyl cyclopentadecanecarboxylate (0.90 g, 2.09 mmol, 48.6% yield) as a colorless oil.

¹H NMR (400 MHz, CHLOROFORM-*d*): *δ* 1.29 - 1.40 (m, 30 H) 1.56 - 1.64 (m, 6 H) 1.87 (quin2 H) 2.34 - 2.46 (m, 1 H) 3.41 (t, 2 H) 4.07 (t, 2 H)

### 5-5. Synthesis of 7-bromoheptyl 4-pentylcyclohexane-1-carboxylate

In a 250 mL 3-neck RBF, 4-pentylcyclohexane-1-carboxylic acid (4.47 g, 22.9 mmol, 1.00 eq) was added together with toluene (50 mL), and 7-bromoheptan-1-ol (5.00 g, 25.2 mmol, 1.00 eq) was added thereto. H₂SO₄ (450 mg, 4.58 mmol, 244 µL, 0.20 eq) was then added thereto, and the resulting mixture was purged three times with nitrogen gas. The resulting mixture was stirred at 120 °C for 16 hours under a nitrogen atmosphere. The reaction mixture was concentrated in vacuo, and the concentrated residue was purified by silica column chromatography (petroleum ether:EtOAc = 10:1) to obtain 7-bromoheptyl 4-pentylcyclohexane-1-carboxylate (7.00 g, 18.7 mmol, 81.4% yield) as a colorless oil.

¹H NMR (400 MHz, CHLOROFORM-*d*): *δ* 0.86 - 0.90 (m, 3 H) 1.18 - 1.32 (m, 10 H) 1.32 - 1.40 (m, 5 H) 1.41 - 1.59 (m, 6 H) 1.59 - 1.68 (m, 2 H) 1.81 - 1.90 (m, 2 H) 1.91 - 2.02 (m, 2 H) 2.46 - 2.54 (m, 1 H) 3.41 (t, 2 H) 4.02 - 4.12 (m, 2 H)

### 5-6. Synthesis of 7-((2-hydroxyethyl)amino)heptyl 4-pentylcyclohexane-1-carboxylate

In a 250 mL 3-neck RBF, 7-bromoheptyl 4-pentylcyclohexane-1-carboxylate (3.00 g, 7.99 mmol, 1.00 eq) and 2-aminoethan-1-ol (2.44 g, 40.0 mmol, 2.41 mL, 5.00 eq) were added together with 1,4-dioxane (90 mL), and the resulting mixture was purged three times with nitrogen gas. The resulting mixture was stirred at 100 °C for 16 hours under a nitrogen atmosphere. The reaction mixture was concentrated in vacuo, and the concentrated residue was purified by silica column chromatography (DCM:MeOH = 10:1) to obtain 7-((2-hydroxyethyl)amino)heptyl 4-pentylcyclohexane-1-carboxylate (1.70 g, 4.78 mmol, 59.8% yield) as a yellow oil.

¹H NMR (400 MHz, CHLOROFORM-*d*): *δ* 0.88 (t, 3 H) 1.19 - 1.28 (m, 8 H) 1.34 (br s, 6 H) 1.41 - 1.71 (m, 8 H) 1.84 - 2.01 (m, 6 H) 2.42 - 2.54 (m, 1 H) 2.63 (t, 2 H) 2.74 - 2.86 (m, 2 H) 3.50 - 3.79 (m, 2 H) 3.99 - 4.13 (m, 2 H)

### 5-7. Synthesis of the compound of Formula E

In a 50 mL 3-neck RBF, 7-((2-hydroxyethyl)amino)heptyl 4-pentylcyclohexane-1-carboxylate (90 mg, 209 µmol, 1.00 eq) was added together with 1,4-dioxane (5 mL), and 7-bromoheptyl cyclopentadecanecarboxylate (74.2 mg, 209 µmol, 1.00 eq) and DIEA (135 mg, 1.04 mmol, 5.00 eq) were added thereto. The resulting mixture was purged three times with nitrogen gas and stirred at 105 °C for 16 hours under a nitrogen atmosphere. The reaction mixture was cooled to 25 °C, and water (10 mL) was added thereto to quench the reaction. The resulting mixture was extracted with EtOAc 30 mL (10 mL x 3). The combined organic layers were dried over Na₂SO₄ and filtered. The filtrate was concentrated in vacuo, and the concentrated residue was purified by silica column chromatography (DCM:MeOH = 10:1) to obtain the compound of Formula E (70 mg, 99.1 µmol, 47.5% yield) as a yellow oil.

¹H NMR (400 MHz, CHLOROFORM-*d*): *δ* 0.88 (t, 3 H) 1.23 - 1.40 (m, 46 H) 1.45 - 1.50 (m, 4 H) 1.52 - 1.66 (m, 12 H) 1.72 - 2.05 (m, 4 H) 2.37 - 2.55 (m, 6 H) 2.63 (br t, 2 H) 3.57 (br t, 2 H) 4.02 - 4.09 (m, 4 H)

### Lipid Preparation Example 6

The compound of the following Formula F was prepared as follows.

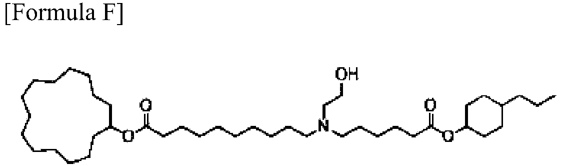

### 6-2. Synthesis of cyclopentadecyl 10-bromodecanoate

The synthesis was performed according to the method described in Preparation Example 3-2.

### 6-3. Synthesis of 4-propylcyclohexyl 6-bromohexanoate

The synthesis was performed according to the method described in Preparation Example 2-3.

### 6-4. Synthesis of 4-propylcyclohexyl 6-((2-hydroxyethyl)amino)hexanoate

The synthesis was performed according to the method described in Preparation Example 2-4.

### 6-5. Synthesis of the compound of Formula F

In a 50 mL 3-neck RBF, 4-propylcyclohexyl 6-((2-hydroxyethyl)amino)hexanoate (0.10 g, 1.00 eq), cyclopentadecyl 10-bromodecanoate (153 mg, 1.00 eq), and DIEA (44 mg, 1.00 eq) were added together with EtOH (5 mL), and the resulting mixture was purged three times with nitrogen gas. The resulting mixture was stirred at 95 °C for 16 hours under a nitrogen atmosphere. The reaction mixture was cooled to room temperature and concentrated in vacuo. The concentrated residue was purified by silica column chromatography (DCM:MeOH = 100:1 → 10:1) to obtain the compound of Formula F (70 mg, 30.9% yield) as a yellow oil.

¹H NMR (400 MHz, CHLOROFORM-*d*): *δ* 5.03 - 4.62 (m, 2H), 4.01 - 3.72 (m, 2H), 3.10 - 2.75 (m, 6H), 2.35 - 2.25 (m, 4H), 1.95 (br d, 1H), 1.59 (br s, 18H), 1.40 - 1.17 (m, 42H), 1.05 - 0.96 (m, 1H), 0.92 - 0.87 (m, 3H)

### Lipid Preparation Example 7

The compound of Formula G shown below was prepared in the same manner as in Preparation Example 1, except that 3-pentylcyclopentan-1-ol was used in place of 4-pentylcyclohexan-1-ol.

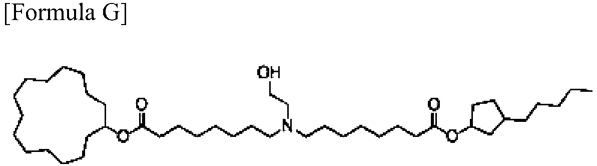

¹H NMR (400 MHz, CHLOROFORM-d): *δ* 5.03 - 4.77 (m, 2H), 4.09 - 3.88 (m, 2H), 3.04 - 2.88 (br, 6H), 2.41 - 2.31 (m, 4H), 1.89 - 1.56 (br, 14H), 1.51 - 0.96 (br, 53H), 0.88 (t, 3H)

### Lipid Preparation Example 8

The compound of Formula H shown below was prepared in the same manner as in Preparation Example 1, except that 6-bromohexanoic acid was used in place of 8-bromooctanoic acid and 3-pentylcyclopentan-1-ol was used in place of 4-pentylcyclohexan-1-ol.

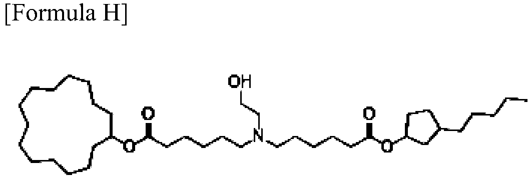

¹H NMR (400 MHz, CHLOROFORM-*d*): *δ* 4.68 (m, 1H), 4.48 (m, 1H), 3.91 (m, 2H), 3.02 - 2.89 (br, 6H), 2.35 - 2.29 (m, 4H), 1.92 - 1.61 (m, 14H), 1.58 - 1.01 (m, 45H), 0.90 (t, 3H)

### Lipid Preparation Example 9

The compound of Formula I shown below was prepared in the same manner as in Preparation Example 1, except that 10-bromodecanoic acid was used in place of 8-bromooctanoic acid and 3-pentylcyclopentan-1-ol was used in place of 4-pentylcyclohexan-1-ol.

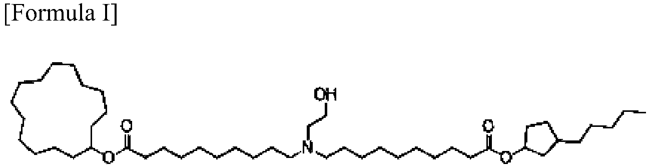

¹H NMR (400 MHz, CHLOROFORM-*d*): *δ* 4.70 (m, 1H), 4.47 (m, 1H), 3.90 (m, 2H), 2.99 - 2.79 (br, 6H), 2.31 - 2.26 (m, 4H), 1.92 - 1.61 (m, 14H), 1.58 - 1.01 (m, 61H), 0.85 (t, 3H)

### Lipid Preparation Example 10

The compound of Formula J shown below was prepared in the same manner as in Preparation Example 1, except that cyclohexanol was used in place of cyclopentadecanol and 3-pentylcyclopentan-1-ol was used in place of 4-pentylcyclohexan-1-ol.

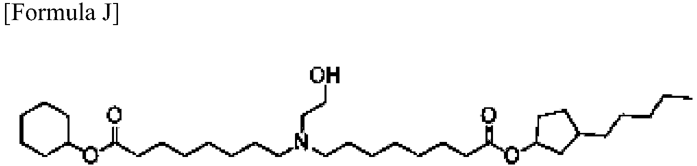

¹H NMR (400 MHz, CHLOROFORM-*d*): *δ* 4.68 - 4.61 (m, 2H), 3.94 (m, 2H), 3.12 - 2.94 (br, 6H), 2.44 (m, 4H), 1.91 - 1.10 (m 45H), 0.88 (t, 3H)

### Lipid Preparation Example 11

The compound of Formula K shown below was prepared in the same manner as in Preparation Example 1, except that aminocyclopentadecane was used in place of cyclopentadecanol.

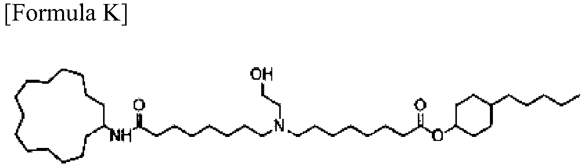

¹H NMR (400 MHz, CHLOROFORM-*d*): *δ* 8.10 (br, 1H), 4.67 - 4.63 (m, 1H), 3.95 - 3.88 (m, 2H), 3.64 - 3.59 (m, 1H), 3.11 - 2.89 (br, 6H), 2.31 - 2.19 (m, 4H), 1.95 - 1.25 (m, 65H), 0.88 (t, 3H)

### Lipid Preparation Example 12

The compound of Formula L shown below was prepared in the same manner as in Preparation Example 1, except that 1-amino-4-pentylcyclohexane was used in place of 4-pentylcyclohexan-1-ol.

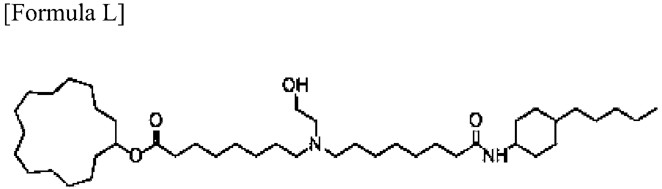

¹H NMR (400 MHz, CHLOROFORM-*d*): *δ* 8.12 (br, 1H), 4.50 - 4.43 (m, 1H), 3.88 - 3.76 (m, 2H), 3.49 - 3.40 (m, 2H), 3.36 - 3.33 (m, 1H), 3.03 - 2.90 (br, 6H), 2.31 - 2.90 (m, 4H), 1.92 - 1.20 (m, 65H), 0.90 (t, 3H)

### Lipid Preparation Example 13

The compound of Formula M shown below was prepared in the same manner as in Preparation Example 1, except that aminocyclopentadecane was used in place of cyclopentadecanol and 1-amino-4-pentylcyclohexane was used in place of 4-pentylcyclohexan-1-ol.

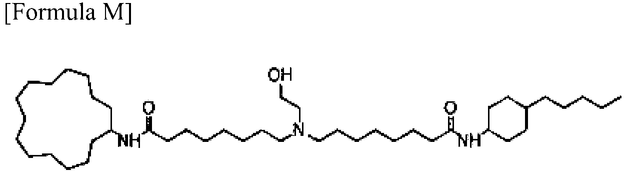

¹H NMR (400 MHz, CHLOROFORM-*d*): *δ* 8.10 (br, 2H), 3.84 - 3.78 (m, 2H), 3.69 - 3.61 (m, 2H), 3.11 - 2.98 (br, 6H), 2.33 - 2.19 (m, 4H), 1.90 - 1.22 (m, 65H), 0.91 (t, 3H)

### Lipid Preparation Example 14

The compound of Formula N shown below was prepared in the same manner as in Preparation Example 1, except that (1R,2R)-2-pentylcyclopropan-1-ol prepared in Example 4-4 was used in place of 4-pentylcyclohexan-1-ol.

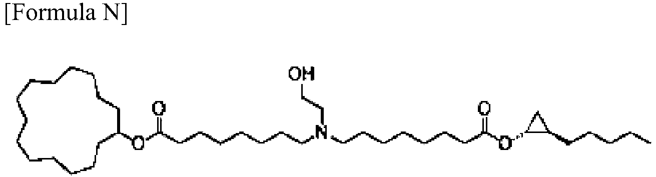

¹H NMR (400 MHz, CHLOROFORM-*d*): *δ* 4.56 - 4.45 (m, 1H), 3.56 - 3.52 (m, 2H), 3.42 (br, 1H), 3.11 - 3.05 (br, 4H), 2.77 - 2.70 (br, 2H), 2.44 - 2.38 (m, 4H), 1.91 - 1.21 (m, 57H), 0.9 (t, 3H), 0.62 - 0.55 (m, 2H)

### Lipid Preparation Example 15

The compound of Formula O shown below was prepared in the same manner as in Preparation Example 1, except that methylamine (CH₃NH₂) was used in place of 2-aminoethan-1-ol.

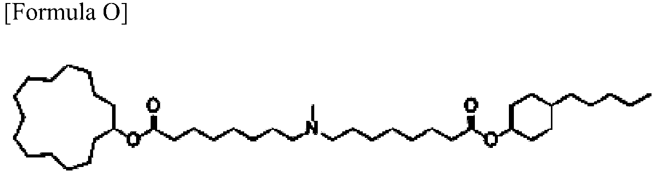

¹H NMR (400 MHz, CHLOROFORM-*d*): *δ* 4.41 - 4.38 (m, 1H), 4.22 - 4.18 (m, 1H), 3.00 - 2.82 (m, 4H), 2.75 (s, 3H), 2.42 - 2.39 (m, 4H), 1.92 - 1.20 (m, 65H), 0.90 (t, 3H)

### Lipid Preparation Example 16

The compound of Formula P shown below was prepared in the same manner as in Preparation Example 1, except that methylamine (CH₃NH₂) was used in place of 2-aminoethan-1-ol, 10-bromodecanoic acid was used in place of 8-bromooctanoic acid, and 4-heptylcyclohexan-1-ol was used in place of 4-pentylcyclohexan-1-ol.

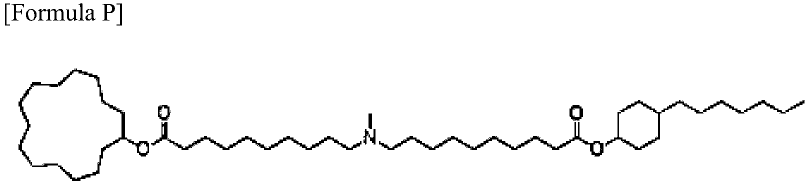

¹H NMR (400 MHz, CHLOROFORM-*d*): *δ* 4.43 - 4.40 (m, 1H), 4.21 - 4.18 (m, 1H), 3.12 - 2.92 (m, 4H), 2.80 (s, 3H), 2.42 - 2.39 (m, 4H), 1.93 - 1.19 (m, 73H), 0.90 (t, 3H)

### Lipid Preparation Example 17

The compound of Formula Q shown below was prepared in the same manner as in Preparation Example 1, except that cyclodecanol was used in place of cyclopentadecanol.

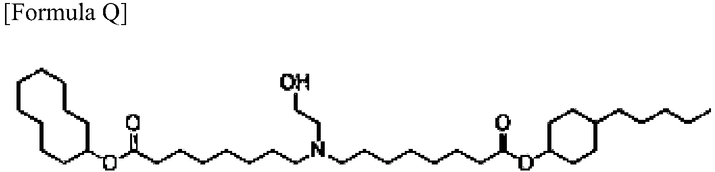

¹H NMR (400 MHz, CHLOROFORM-*d*): *δ* 4.48 - 4.41 (m, 1H), 4.19 - 4.17 (m, 1H), 3.54 - 3.49 (m, 2H), 3.02 - 2.92 (br, 4H), 2.78 - 2.71 (br, 2H), 2.35 - 2.31 (m, 4H), 1.91 - 1.19 (m, 55H), 0.88 (t, 3H)

### Lipid Preparation Example 18

The compound of Formula R shown below was prepared in the same manner as in Preparation Example 1, except that cyclodecanol was used in place of cyclopentadecanol and methylamine (CH₃-NH₂) was used in place of 2-aminoethan-1-ol.

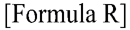

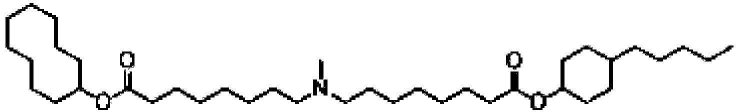

¹H NMR (400 MHz, CHLOROFORM-*d*): *δ* 4.48 - 4.41 (m, 1H), 4.19 - 4.17 (m, 1H), 3.01 - 2.93 (br, 4H), 2.75 (br, 3H), 2.34 - 2.30 (m, 4H), 1.90 - 1.21 (m, 55H), 0.90 (t, 3H)

### [Preparation of compositions and test of delivery of effective ingredient to tissue]

### Example 1: Preparation of composition for drug delivery using lipid-polymer and each lipid of Preparation Examples 1, 2, 3, 5 or 6, and test of drug delivery

### (1) Preparation of solutions for each component

The components shown in Table 1 below were dissolved in the respective dilution solvents and prepared at the concentrations shown in Table 1 below. After dissolution at the appropriate concentrations, the absence of any undissolved precipitate was confirmed by visual inspection, and the resulting solutions were used for the preparation of the compositions.

**[Table 1]**

| No. | Components | Dilution solvents | Concentration for use |
|---|---|---|---|
| 1 | mRNA | RNAse Free water | 1 mg/mL |
| 2 | Each lipid of Preparation Examples 1, 2, 3, 5 or 6 | 100% ethanol | 10-20 mg/mL |
| 3 | DOPE(1,2-dioleoyl-sn-glycero-3-phosphoethanolamine) or DSPC(1,2-distearoyl-sn-glycero-3-phosphocholine) | 100% ethanol | 10-20 mg/mL |
| 4 | Cholesterol | 100% ethanol | 10-20 mg/mL |
| 5 | DMG-PEG (1,2-dimyristoyl-rac-glycero-3-methoxypolyethylene glycol-2000) | 100% ethanol | 5-20 mg/mL |

### (2) Mixing of raw materials

The components were taken and mixed according to the weight ratio of each lipid of Preparation Examples 1, 2, 3, 5 or 6: DOPE or DSPC: cholesterol: DMG-PEG shown in Table 2 below, while maintaining an N/P ratio (amine groups of the lipid components/phosphate groups of the mRNA) of 6. Ethanol was additionally added to the ethanol phase such that the molecular total of all components was 6.25-12.5 mM, and the aqueous phase and the ethanol phase were mixed while maintaining a ratio of 3:1. After mixing, buffer exchange was performed as follows to reduce the overall ethanol content: The mixture was subjected to buffer exchange by concentration and dilution with PBS using an Amicon Ultra tube filter (Merck Millipore, UFC505096 or UFC805024; pore size: 50K or 100K; volume: 0.5 mL, 4 mL, or 15 mL) and centrifugation at 4,000 rpm.

The specific process was carried out as follows:
1) Two autoclaved tubes were prepared (Tubes (A) and (B)).
2) To Tube (A), each lipid of Preparation Examples 1, 2, 3, 5 or 6, DOPE or DSPC, cholesterol, and DMG-PEG were sequentially added in amounts corresponding to the numbers of moles calculated according to the experimental conditions, and mixed by vortexing.
3) Ethanol was added to the ethanol phase, as necessary, such that the total molar concentration of all components was within 6.25-12.5 mM.
4) mRNA and 20 mM sodium acetate buffer (pH 4.6) were mixed in Tube (B). At this time, the components were added such that the volume of the aqueous phase was three times the total volume of the ethanol phase (aqueous phase:ethanol phase volume ratio = 3:1).
5) The contents of Tube (A) and Tube (B) were mixed.
6) The mixture obtained in step 5) was centrifuged at 4,000 rpm using an Amicon-Ultra tube filter (50K), and excess ethanol was removed by repeating concentration and dilution cycles. The mixture was then finally concentrated to an intended concentration of x mg/mL (theoretical concentration).
7) After the desired concentration was reached, the formulation was sterilized using a 0.22 µm pore size filter.

**[Table 2]**

| | Effective ingredient | Lipid | Fusogenic lipid | | Polymer |
|---|---|---|---|---|---|
| Comparative Example | mRNA | SM102 | DSPC | Cholesterol | DMG-PEG |
| 1 | 1 mg | 11.4 mg | 2.5 mg | 4.8 mg | 1.2 mg |
| Example | mRNA | Preparation Example 1 | DOPE | Cholesterol | DMG-PEG |
| 1-1 | 1 mg | 13.1 mg | 4.6 mg | 14.0 mg | 2.3 mg |
| 1-2 | 1 mg | 13.1 mg | 13.8 mg | 9.2 mg | 2.3 mg |
| 1-3 | 1 mg | 13.1 mg | 3.5 mg | 8.7 mg | 1.7 mg |
| 1-4 | 1 mg | 13.1 mg | 10.4 mg | 5.1 mg | 1.7 mg |
| 1-5 | 1 mg | 13.1 mg | 5.5 mg | 4.1 mg | 1.4 mg |
| 1-6 | 1 mg | 13.1 mg | 8.3 mg | 2.7 mg | 1.4 mg |
| 1-7 | 1 mg | 13.1 mg | 4.6 mg | 2.2 mg | 1.2 mg |
| 1-8 | 1 mg | 13.1 mg | 2.0 mg | 1.9 mg | 1.0 mg |
| 1-9 | 1 mg | 13.1 mg | 2.8 mg | 5.5 mg | 1.4 mg |
| Example | mRNA | Preparation Example 2/3/5/6 | DOPE/DSPC | Cholesterol | DMG-PEG |
| 1-10 | 1 mg | Preparation Example 3: 14.7 mg | DOPE: 2.8 mg | 5.5 mg | 1.4 mg |
| 1-11 | 1 mg | Preparation Example 5: 13.1 mg | DOPE: 2.8 mg | 5.5 mg | 1.4 mg |
| 1-12 | 1 mg | Preparation Example 6: 12.6 mg | DOPE: 2.8 mg | 5.5 mg | 1.4 mg |
| 1-13 | 1 mg | Preparation Example 2: 11.6 mg | DOPE: 2.8 mg | 5.5 mg | 1.4 mg |
| 1-14 | 1 mg | Preparation Example 3: 14.7 mg | DSPC: 2.9 mg | 5.5 mg | 1.4 mg |
| 1-15 | 1 mg | Preparation Example 5: 13.1 mg | DSPC: 2.9 mg | 5.5 mg | 1.4 mg |
| 1-16 | 1 mg | Preparation Example 6: 12.6 mg | DSPC: 2.9 mg | 5.5 mg | 1.4 mg |
| 1-17 | 1 mg | Preparation Example 2: 11.6 mg | DSPC: 2.9 mg | 5.5 mg | 1.4 mg |

| | | | | | |
|---|---|---|---|---|---|
| SM102: Heptadecan-9-yl 8-((2-hydroxyethyl) (6-oxo-6-(undecyloxy)hexyl)amino) octanoate (SINOPEG) | | | | | |

### (3) Evaluation of formulation properties

1) The particle characteristics of the prepared formulations were analyzed using a particle size analyzer based on dynamic light scattering (DLS), and the results are shown in Table 3 below.
2) The encapsulation efficiency of mRNA in the prepared formulations was evaluated using a RiboGreen assay, and the results are shown in Table 3 below.

### (4) Administration of composition

The prepared formulations were adjusted to a concentration of 10 µg/mL and intravenously administered (intravascular administration) to mice so that 2 µg of mRNA per mouse was administered. 4 hours after administration, luciferin dissolved in sterile water was prepared at 15 µg/µL and intraperitoneally administered so that 3 mg of luciferin per 20 g mouse was delivered. 15 minutes after intraperitoneal administration of luciferin, protein expression in each organ was measured using a bioluminescence imaging system, and the results are shown in Table 3 below.

As can be seen from Table 3, the drug delivery formulations according to the present invention exhibited excellent efficiency of drug delivery to target organ upon administration.

Meanwhile, the formulation of Comparative Example 1 was intravenously administered to mice at the same mRNA dose (2 µg/200 µL) as in the Examples, and imaging was performed 4 hours later in the same manner as in the Examples, with the results shown in Table 3. The comparative formulation showed delivery to the target organ upon administration.

**[Table 3]**

| | Zeta-average (nm) | PD index (PI) | Zeta-potential (mV) | Encapsulation efficiency (%) | Liver Avg Radiance [p/s/cm²/sr] |
|---|---|---|---|---|---|
| Comparative Example 1 | 105.8 ± 0.96 | 0.09 ± 0.02 | -3.24 ± 2.72 | 97.8 | 1.02E+08 |
| Example 1-1 | 151.7 ± 0.95 | 0.17 ± 0.03 | -10.41 ± 3.18 | 96.8 | 3.84E+07 |
| Example 1-2 | 163.7 ± 2.47 | 0.14 ± 0.02 | -3.34 ± 3.55 | 96.4 | 2.07E+07 |
| Example 1-3 | 161.7 ± 1.61 | 0.14 ± 0.05 | -9.26 ± 2.54 | 97.5 | 1.05E+08 |
| Example 1-4 | 137.5 ± 2.59 | 0.13 ± 0.07 | -12.82 ± 3.52 | 96.5 | 4.50E+07 |
| Example 1-5 | 200.0 ± 6.19 | 0.20 ± 0.05 | -6.56 ± 5.37 | 94.6 | 2.86E+07 |
| Example 1-6 | 140.3 ± 2.95 | 0.13 ± 0.02 | -1.18 ± 6.70 | 93.3 | 4.19E+06 |
| Example 1-7 | 177.9 ± 8.18 | 0.23 ± 0.01 | -8.69 ± 5.81 | 94.2 | 3.87E+06 |
| Example 1-8 | 145.6 ± 1.11 | 0.13 ± 0.02 | -9.03 ± 9.50 | 90.5 | 3.83E+06 |
| Example 1-9 | 129.9 ± 3.12 | 0.07 ± 0.02 | -0.84 ± 5.47 | 94.0 | 1.10E+08 |
| Example 1-10 | 144.5 ± 3.12 | 0.14 ± 0.01 | -3.36 ± 1.43 | 99.0 | 7.97E+07 |
| Example 1-11 | 121.1 ± 1.25 | 0.09 ± 0.00 | -3.36 ± 2.09 | 99.9 | 6.68E+07 |
| Example 1-12 | 155.0 ± 2.85 | 0.08 ± 0.03 | -5.80 ± 0.88 | 99.7 | 4.55E+07 |
| Example 1-13 | 153.5 ± 2.81 | 0.17 ± 0.02 | -7.54 ± 1.15 | 99.7 | 1.26E+06 |
| Example 1-14 | 120.0 ± 3.11 | 0.07 ± 0.01 | -3.81 ± 0.54 | 97.8 | 1.34E+08 |
| Example 1-15 | 136.3 ± 1.25 | 0.12 ± 0.00 | -6.08 ± 2.30 | 99.9 | 7.14E+07 |
| Example 1-16 | 149.5 ± 2.85 | 0.22 ± 0.03 | -5.63 ± 2.33 | 99.7 | 1.97E+07 |
| Example 1-17 | 152.0 ± 2.81 | 0.12 ± 0.02 | -7.5 ± 0.33 | 99.7 | 1.23E+06 |

### Example 2: Preparation of composition for drug delivery using amphiphilic block copolymer and the lipid of Preparation Example 1, and test of drug delivery

### (1) Preparation of solutions for each component

The components shown in Table 4 below were used to prepare solutions of each component at the concentrations shown in Table 4 below, in the same manner as in step (1) of Example 1.

**[Table 4]**

| No. | Components | Dilution solvents | Concentration for use |
|---|---|---|---|
| 1 | mRNA | RNAse Free water | 1 mg/mL |
| 2 | Lipid of Preparation Example 1 | 100% ethanol | 10-20 mg/mL |
| 3 | DOPE or DSPC | 100% ethanol | 10-20 mg/mL |
| 4 | Cholesterol | 100% ethanol | 10-20 mg/mL |
| 5 | MPEG-PLA(2K-4K) | 95% ethanol | 50-100 mg/mL |

| | | | |
|---|---|---|---|
| mPEG-PLA(2K-4K): Copolymer of a monomethoxypolyethylene glycol (mPEG) block with a number average molecular weight of 2,000 and a polylactic acid (PLA) block with a number average molecular weight of 4,000 | | | |

### (2) Mixing of raw materials

The components were taken and mixed according to the weight ratio of the lipid of Preparation Example 1: DOPE: cholesterol: DMG-PEG shown in Table 5 below, while maintaining an N/P ratio (amine groups of the lipid components/phosphate groups of the mRNA) of 6-20. Ethanol was additionally added to the ethanol phase such that the molecular total of all components was 6.25-12.5 mM, and the aqueous phase and the ethanol phase were mixed while maintaining a ratio of 3:1. After mixing, buffer exchange was performed as follows to reduce the overall ethanol content: The mixture was subjected to buffer exchange by concentration and dilution with PBS using an Amicon Ultra tube filter (Merck Millipore, UFC505096 or UFC805024; pore size: 50K or 100K; volume: 0.5 mL, 4 mL, or 15 mL) and centrifugation at 4,000 rpm.

The specific process was carried out as follows:
1) Two autoclaved tubes were prepared (Tubes (A) and (B)).
2) To Tube (A), the lipid of Preparation Example 1, DSPC, cholesterol, and MPEG-PLA(2K-4K) were sequentially added in amounts corresponding to the numbers of moles calculated according to the experimental conditions, and mixed by vortexing.
3) Ethanol was added to the ethanol phase, as necessary, such that the total molar concentration of all components was within 6.25-12.5 mM.
4) mRNA and sodium acetate buffer with a pH adjusted to 4.6 were mixed in Tube (B). At this time, the components were added such that the volume of the aqueous phase was three times the total volume of the ethanol phase (aqueous phase:ethanol phase volume ratio = 3:1).
5) The contents of Tube (A) and Tube (B) were mixed.
6) The mixture obtained in step 5) was centrifuged at 4,000 rpm using an Amicon-Ultra tube filter (50K), and excess ethanol was removed by repeating concentration and dilution cycles. The mixture was then finally concentrated to an intended concentration of x mg/mL (theoretical concentration).
7) After the desired concentration was reached, the formulation was sterilized using a 0.22 µm pore size filter.

**[Table 5]**

| | Effective ingredient | Lipid | Fusogenic lipid | | Polymer |
|---|---|---|---|---|---|
| Comparative Example | mRNA | Lipid5/SM102 | DSPC | Cholesterol | DMG-PEG |
| 2-1 | 1 mg | Lipid5: 11.1 mg | 2.5 mg | 4.6 mg | 1.2 mg |
| 2-2 | 1 mg | SM102: 11.1 mg | 2.5 mg | 4.6 mg | 1.2 mg |
| Example | mRNA | Preparation Example 1 | DOPE | Cholesterol | MPEG-PLA(2K-4K) |
| 2 | 1 mg | 12.7 mg | 2.7 mg | 5.6 mg | 108.1 mg |

| | | | | | |
|---|---|---|---|---|---|
| Lipid5: 8-[(2-hydroxyethyl)[8-(nonyloxy)-8-oxooctyl]amino]-octanoic acid, 1-octylnonyl ester | | | | | |

### (3) Evaluation of formulation properties

The properties of the prepared formulations were evaluated in the same manner as in step (3) of Example 1, and the results are shown in Table 6 below.

### (4) Administration of composition

The prepared formulations were adjusted to a concentration of 10 µg/mL and intravenously administered (intravascular administration) to mice so that 2 µg of mRNA per mouse was administered. 4 hours after administration, luciferin dissolved in sterile water was prepared at 15 µg/µL and intraperitoneally administered so that 3 mg of luciferin per 20 g mouse was delivered. 15 minutes after intraperitoneal administration of luciferin, protein expression in each organ was measured using a bioluminescence imaging system, and the results are shown in Table 6 below.

As can be seen from Table 6, the drug delivery formulation according to the present invention exhibited excellent efficiency of drug delivery to target organ upon administration.

Meanwhile, each of the formulations of Comparative Examples 2-1 and 2-2 was intravenously administered to mice at the same mRNA dose (2 µg/200 µL) as in the Examples, and imaging was performed 4 hours later in the same manner as in the Examples, with the results shown in Table 6. The comparative formulations showed delivery to the target organ upon administration.

**[Table 6]**

| | Zeta-average (nm) | PD index (PI) | Zeta-potential (mV) | Encapsulation efficiency (%) | Liver Avg Radiance [p/s/cm²/sr] |
|---|---|---|---|---|---|
| Comparative Example 2-1 | 103.7 ± 0.99 | 0.13 ± 0.02 | -6.52 ± 1.18 | 90.3 | 2.0.E+07 |
| Comparative Example 2-2 | 101.0 ± 0.5 | 0.09 ± 0.01 | -5.47 ± 1.55 | 95.1 | 2.0.E+08 |
| Example 2 | 132.7 ± 2.2 | 0.14 ± 0.02 | -4.46 ± 1.32 | 98.3 | 4.00E+07 |

### Example 3: Preparation of composition for drug delivery using amphiphilic block copolymer and the lipid of Preparation Example 3, and test of drug delivery

### (1) Preparation of solutions for each component

The components shown in Table 7 below were used to prepare solutions of each component at the concentrations shown in Table 7 below, in the same manner as in step (1) of Example 1.

**[Table 7]**

| No. | Components | Dilution solvents | Concentration for use |
|---|---|---|---|
| 1 | mRNA | RNAse Free water | 1 mg/mL |
| 2 | Lipid of Preparation Example 3 | 100% ethanol | 10-20 mg/mL |
| 3 | DOPE or DSPC | 100% ethanol | 10-20 mg/mL |
| 4 | Cholesterol | 100% ethanol | 10-20 mg/mL |
| 5 | MPEG-PLA(2K-4K) | 95% ethanol | 50-100 mg/mL |

### (2) Mixing of raw materials

The components were taken and mixed according to the weight ratio of the lipid of Preparation Example 3: DOPE or DSPC: cholesterol: MPEG-PLA(2K-4K) shown in Table 8 below, while maintaining an N/P ratio (amine groups of the lipid components/phosphate groups of the mRNA) of 6. Ethanol was additionally added to the ethanol phase such that the molecular total of all components was 6.25-12.5 mM, and the aqueous phase and the ethanol phase were mixed while maintaining a ratio of 3:1. After mixing, buffer exchange was performed as follows to reduce the overall ethanol content: The mixture was subjected to buffer exchange by concentration and dilution with PBS using an Amicon Ultra tube filter (Merck Millipore, UFC505096 or UFC805024; pore size: 50K or 100K; volume: 0.5 mL, 4 mL, or 15 mL) and centrifugation at 4,000 rpm.

The specific process was carried out as follows:
1) Two autoclaved tubes were prepared (Tubes (A) and (B)).
2) To Tube (A), the lipid of Preparation Example 3, DOPE or DSPC, cholesterol, and MPEG-PLA(2K-4K) were sequentially added in amounts corresponding to the numbers of moles calculated according to the experimental conditions, and mixed by vortexing.
3) Ethanol was added to the ethanol phase, as necessary, such that the total molar concentration of all components was within 6.25-12.5 mM.
4) mRNA and 20 mM sodium acetate buffer (pH 4.6) were mixed in Tube (B). At this time, the components were added such that the volume of the aqueous phase was three times the total volume of the ethanol phase (aqueous phase:ethanol phase volume ratio = 3:1).
5) The contents of Tube (A) and Tube (B) were mixed.
6) The mixture obtained in step 5) was centrifuged at 4,000 rpm using an Amicon-Ultra tube filter (50K), and excess ethanol was removed by repeating concentration and dilution cycles. The mixture was then finally concentrated to an intended concentration of x mg/mL (theoretical concentration).
7) After the desired concentration was reached, the formulation was sterilized using a 0.22 µm pore size filter.

**[Table 8]**

| | Effective ingredient | Lipid | Fusogenic lipid | | Polymer |
|---|---|---|---|---|---|
| Comparative Example | mRNA | Lipid5 | DSPC | Cholesterol | DMG-PEG |
| 3-1 | 1 mg | 11.1 mg | 2.5 mg | 4.6 mg | 1.2 mg |
| Example | mRNA | Preparation Example 3 | DOPE/DSPC | Cholesterol | MPEG-PLA(2K-4K) |
| 3-1 | 1 mg | 14.7 mg | DOPE: 2.8 mg | 5.7 mg | 111.5 mg |
| 3-2 | 1 mg | 14.7 mg | DSPC: 2.9 mg | 5.7 mg | 111.5 mg |

### (3) Evaluation of formulation properties

The properties of the prepared formulations were evaluated in the same manner as in step (3) of Example 1, and the results are shown in Table 9 below.

### (4) Administration of composition

The prepared formulations were adjusted to a concentration of 10 µg/mL and intravenously administered (intravascular administration, IV) to mice so that 2 µg of mRNA per mouse was administered. 4 hours after administration, luciferin dissolved in sterile water was prepared at 15 µg/µL and intraperitoneally administered so that 3 mg of luciferin per 20 g mouse was delivered. 15 minutes after intraperitoneal administration of luciferin, protein expression in each organ was measured using a bioluminescence imaging system, and the results are shown in Table 9 below.

As can be seen from Table 9, the drug delivery formulations according to the present invention exhibited excellent efficiency of drug delivery to target organ upon administration.

Meanwhile, the formulation of Comparative Example 3-1 was intravenously administered to mice at the same mRNA dose (2 µg/200 µL) as in the Examples, and imaging was performed 4 hours later in the same manner as in the Examples, with the results shown in Table 9. The comparative formulation showed delivery to the target organ upon administration.

**[Table 9]**

| | Zeta-average (nm) | PD index (PI) | Zeta-potential (mV) | Encapsulation efficiency (%) | Liver Avg Radiance [p/s/cm²/sr] |
|---|---|---|---|---|---|
| Comparative Example 3-1 | 75.16 ± 1.2 | 0.20 ± 0.00 | -10.95 ± 0.65 | 95.4 | 1.2.E+06 |
| Example 3-1 | 120.8 ± 2.39 | 0.22 ± 0.00 | -3.44 ± 2.36 | 96.6 | 1.1.E+07 |
| Example 3-2 | 122.3 ± 0.71 | 0.13 ±0 .00 | 0.59 ± 2.55 | 69.7 | 1.8.E+07 |

### Example 4: Preparation of composition for drug delivery using amphiphilic block copolymer and the lipid of Preparation Example 5, and test of drug delivery

### (1) Preparation of solutions for each component

The components shown in Table 10 below were used to prepare solutions of each component at the concentrations shown in Table 10 below, in the same manner as in step (1) of Example 1.

**[Table 10]**

| No. | Components | Dilution solvents | Concentration for use |
|---|---|---|---|
| 1 | mRNA | RNAse Free water | 1 mg/mL |
| 2 | Lipid of Preparation Example 5 | 100% ethanol | 10-20 mg/mL |
| 3 | DOPE or DSPC | 100% ethanol | 10-20 mg/mL |
| 4 | Cholesterol | 100% ethanol | 10-20 mg/mL |
| 5 | MPEG-PLA(2K-4K) | 95% ethanol | 50-100 mg/mL |

### (2) Mixing of raw materials

The components were taken and mixed according to the weight ratio of the lipid of Preparation Example 5: DOPE: cholesterol: MPEG-PLA(2K-4K) shown in Table 11 below, while maintaining an N/P ratio (amine groups of the lipid components/phosphate groups of the mRNA) of 6. Ethanol was additionally added to the ethanol phase such that the molecular total of all components was 6.25-12.5 mM, and the aqueous phase and the ethanol phase were mixed while maintaining a ratio of 3:1. After mixing, buffer exchange was performed as follows to reduce the overall ethanol content: The mixture was subjected to buffer exchange by concentration and dilution with PBS using an Amicon Ultra tube filter (Merck Millipore, UFC505096 or UFC805024; pore size: 50K or 100K; volume: 0.5 mL, 4 mL, or 15 mL) and centrifugation at 4,000 rpm.

The specific process was carried out as follows:
1) Two autoclaved tubes were prepared (Tubes (A) and (B)).
2) To Tube (A), the lipid of Preparation Example 5, DOPE, cholesterol, and MPEG-PLA(2K-4K) were sequentially added in amounts corresponding to the numbers of moles calculated according to the experimental conditions, and mixed by vortexing.
3) Ethanol was added to the ethanol phase, as necessary, such that the total molar concentration of all components was within 6.25-12.5 mM.
4) mRNA and 20 mM sodium acetate buffer (pH 4.6) were mixed in Tube (B). At this time, the components were added such that the volume of the aqueous phase was three times the total volume of the ethanol phase (aqueous phase:ethanol phase volume ratio = 3:1).
5) The contents of Tube (A) and Tube (B) were mixed.
6) The mixture obtained in step 5) was centrifuged at 4,000 rpm using an Amicon-Ultra tube filter (50K), and excess ethanol was removed by repeating concentration and dilution cycles. The mixture was then finally concentrated to an intended concentration of x mg/mL (theoretical concentration).
7) After the desired concentration was reached, the formulation was sterilized using a 0.22 µm pore size filter.

**[Table 11]**

| | Effective ingredient | Lipid | Fusogenic lipid | | Polymer |
|---|---|---|---|---|---|
| Comparative Example | mRNA | Lipid5/SM102 | DSPC | Cholesterol | DMG-PEG |
| 4-1 | 1 mg | Lipid5: 11.1 mg | 2.5 mg | 4.6 mg | 1.2 mg |
| 4-2 | 1 mg | SM102: 11.1 mg | 2.5 mg | 4.6 mg | 1.2 mg |

| Example | mRNA | Preparation Example 5 | DOPE | Cholesterol | MPEG-PLA(2K-4K) |
|---|---|---|---|---|---|
| 4-1 | 1 mg | 12.7 mg | 1.9 mg | 2.0 mg | 77.2 mg |
| 4-2 | 1 mg | 12.7 mg | 2.2 mg | 3.5 mg | 90.1 mg |

### (3) Evaluation of formulation properties

The properties of the prepared formulations were evaluated in the same manner as in step (3) of Example 1, and the results are shown in Table 12 below.

### (4) Administration of composition

The prepared formulations were adjusted to a concentration of 10 µg/mL and intravenously administered (intravascular administration, IV) to mice so that 2 µg of mRNA per mouse was administered. 4 hours after administration, luciferin dissolved in sterile water was prepared at 15 µg/µL and intraperitoneally administered so that 3 mg of luciferin per 20 g mouse was delivered. 15 minutes after intraperitoneal administration of luciferin, protein expression in each organ was measured using a bioluminescence imaging system, and the results are shown in Table 12 below.

As can be seen from Table 12, the drug delivery formulations according to the present invention exhibited excellent efficiency of drug delivery to target organ upon administration.

Meanwhile, each of the formulations of Comparative Examples 4-1 and 4-2 was intravenously administered to mice at the same mRNA dose (2 µg/200 µL) as in the Examples, and imaging was performed 4 hours later in the same manner as in the Examples, with the results shown in Table 12. The comparative formulations showed delivery to the target organ upon administration.

**[Table 12]**

| | Zeta-average (nm) | PD index (PI) | Zeta-potential (mV) | Encapsulation efficiency (%) | Liver Avg Radiance [p/s/cm²/sr] |
|---|---|---|---|---|---|
| Comparative Example 4-1 | 139.9 ± 1.11 | 0.08 ± 0.04 | -2.37 ± 1.62 | 90.2 | 5.00E+07 |
| Comparative Example 4-2 | 167.6 ± 0.90 | 0.10 ± 0.02 | -2.50 ± 1.65 | 85.1 | 3.00E+07 |
| Example 4-1 | 234.0 ± 3.05 | 0.31 ± 0.05 | -6.72 ± 1.17 | 90.1 | 5.00E+06 |
| Example 4-2 | 206.9 ± 0.69 | 0.17 ± 0.01 | -2.57 ± 1.96 | 96.3 | 1.0.E+07 |

### Example 5: Preparation of composition for drug delivery using amphiphilic block copolymer and the lipid of Preparation Example 6, and test of drug delivery

### (1) Preparation of solutions for each component

The components shown in Table 13 below were used to prepare solutions of each component at the concentrations shown in Table 13 below, in the same manner as in step (1) of Example 1.

**[Table 13]**

| No. | Components | Dilution solvents | Concentration for use |
|---|---|---|---|
| 1 | mRNA | RNAse Free water | 1 mg/mL |
| 2 | Lipid of Preparation Example 6 | 100% ethanol | 10-20 mg/mL |
| 3 | DOPE or DSPC | 100% ethanol | 10-20 mg/mL |
| 4 | Cholesterol | 100% ethanol | 10-20 mg/mL |
| 5 | MPEG-PLA(2K-4K) | 95% ethanol | 50-100 mg/mL |

### (2) Mixing of raw materials

The components were taken and mixed according to the weight ratio of the lipid of Preparation Example 6: DOPE or DSPC: cholesterol: MPEG-PLA(2K-4K) shown in Table 14 below, while maintaining an N/P ratio (amine groups of the lipid components/phosphate groups of the mRNA) of 6. Ethanol was additionally added to the ethanol phase such that the molecular total of all components was 6.25-12.5 mM, and the aqueous phase and the ethanol phase were mixed while maintaining a ratio of 3:1. After mixing, buffer exchange was performed as follows to reduce the overall ethanol content: The mixture was subjected to buffer exchange by concentration and dilution with PBS using an Amicon Ultra tube filter (Merck Millipore, UFC505096 or UFC805024; pore size: 50K or 100K; volume: 0.5 mL, 4 mL, or 15 mL) and centrifugation at 4,000 rpm.

The specific process was carried out as follows:
1) Two autoclaved tubes were prepared (Tubes (A) and (B)).
2) To Tube (A), the lipid of Preparation Example 6, DOPE or DSPC, cholesterol, and MPEG-PLA(2K-4K) were sequentially added in amounts corresponding to the numbers of moles calculated according to the experimental conditions, and mixed by vortexing.
3) Ethanol was added to the ethanol phase, as necessary, such that the total molar concentration of all components was within 6.25-12.5 mM.
4) mRNA and 20 mM sodium acetate buffer (pH 4.6) were mixed in Tube (B). At this time, the components were added such that the volume of the aqueous phase was three times the total volume of the ethanol phase (aqueous phase:ethanol phase volume ratio = 3:1).
5) The contents of Tube (A) and Tube (B) were mixed.
6) The mixture obtained in step 5) was centrifuged at 4,000 rpm using an Amicon-Ultra tube filter (50K), and excess ethanol was removed by repeating concentration and dilution cycles. The mixture was then finally concentrated to an intended concentration of x mg/mL (theoretical concentration).
7) After the desired concentration was reached, the formulation was sterilized using a 0.22 µm pore size filter.

**[Table 14]**

| | Effective ingredient | Lipid | Fusogenic lipid | | Polymer |
|---|---|---|---|---|---|
| Comparative Example | mRNA | Lipid5 | DSPC | Cholesterol | DMG-PEG |
| 5-1 | 1 mg | 11.1 mg | 2.5 mg | 4.6 mg | 1.2 mg |

| Example | mRNA | Preparation Example 6 | DOPE/DSPC | Cholesterol | MPEG-PLA(2K-4K) |
|---|---|---|---|---|---|
| 5-1 | 1 mg | 12.6 mg | DOPE: 2.8 mg | 5.7 mg | 111.5 mg |
| 5-2 | 1 mg | 12.6 mg | DSPC: 2.9 mg | 5.7 mg | 111.5 mg |

### (3) Evaluation of formulation properties

The properties of the prepared formulations were evaluated in the same manner as in step (3) of Example 1, and the results are shown in Table 15 below.

### (4) Administration of composition

The prepared formulations were adjusted to a concentration of 10 µg/mL and intravenously administered (intravascular administration, IV) to mice so that 2 µg of mRNA per mouse was administered. 4 hours after administration, luciferin dissolved in sterile water was prepared at 15 µg/µL and intraperitoneally administered so that 3 mg of luciferin per 20 g mouse was delivered. 15 minutes after intraperitoneal administration of luciferin, protein expression in each organ was measured using a bioluminescence imaging system, and the results are shown in Table 15 below.

As can be seen from Table 15, the drug delivery formulations according to the present invention exhibited excellent efficiency of drug delivery to target organ upon administration.

Meanwhile, the formulation of Comparative Example 5-1 was intravenously administered to mice at the same mRNA dose (2 µg/200 µL) as in the Examples, and imaging was performed 4 hours later in the same manner as in the Examples, with the results shown in Table 15. The comparative formulation showed delivery to the target organ upon administration.

**[Table 15]**

| | Zeta-average (nm) | PD index (PI) | Zeta-potential (mV) | Encapsulation efficiency (%) | Liver Avg Radiance [p/s/cm²/sr] |
|---|---|---|---|---|---|
| Comparative Example 5-1 | 75.16 ± 1.2 | 0.20 ± 0.00 | -10.95 ± 0.65 | 95.4 | 1.2.E+06 |
| Example 5-1 | 168.4 ± 2.67 | 0.17 ± 0.00 | -8.89 ± 1.21 | 85.2 | 1.1.E+07 |
| Example 5-2 | 939.1 ± 257.2 | 0.85 ± 0.14 | -10.14 ± 1.78 | 23.1 | 2.7.E+05 |

## Claims

1. A composition for drug delivery comprising:
effective ingredient selected from nucleic acid, polypeptide, virus or combination thereof;
a lipid having a structure represented by the following Formula 1; and
lipid-polymer, amphiphilic block copolymer, or a combination thereof: 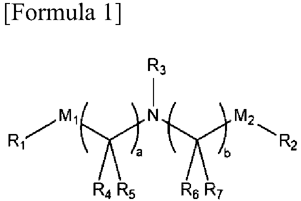
wherein, in the above Formula 1,
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₇ is independently hydrogen atom, or a substituted or unsubstituted, saturated or unsaturated hydrocarbon group, and
each of a and b is independently an integer of from 1 to 20.

2. The composition for drug delivery according to claim 1, wherein
each of M₁ and M₂ is independently selected from the group consisting of -C(O)O-, - OC(O)-, -OC(O)-M'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, arylene, and heteroarylene, wherein M' is a direct bond, C₁₋₁₃ alkylene or C₂₋₁₃ alkenylene, and each R' is independently selected from the group consisting of hydrogen atom, C₁₋₁₈ alkyl and C₂₋₁₈ alkenyl,
each of R₁ and R₂ is independently selected from the group consisting of C₃₋₂₀ cycloalkyl, C₃₋₂₀ cycloalkenyl, C₆₋₂₀ aryl, C₃₋₂₀ heterocycloalkyl, C₃₋₂₀ heterocycloalkenyl, and C₃₋₂₀ heteroaryl, each of which is independently unsubstituted or substituted with C₁₋₁₈ alkyl or C₂₋₁₈ alkenyl,
R₃ is selected from the group consisting of hydrogen atom, substituted or unsubstituted C₁₋₆ alkyl, substituted or unsubstituted C₃₋₆ carbocyclic group, -(CH₂)ₙQ, - (CH₂)ₙCHQR, -CHQR and -CQ(R)₂, wherein each R is independently selected from the group consisting of hydrogen atom, C₁₋₃ alkyl and C₂₋₃ alkenyl; Q is selected from the group consisting of carbocyclic group, heterocyclic group, -OR, -O(CH₂)ₙN(R)₂, -C(O)OR, -OC(O)R, -CX₃, -CX₂H, -CXH₂, -CN, -N(R)₂, -C(O)N(R)₂, -N(R)C(O)R, -N(R)S(O)₂R, -N(R)C(O)N(R)₂, -N(R)C(S)N(R)₂, -N(R)R₁₂, N(R)S(O)₂R₁₂, -O(CH₂)ₙOR, -N(R)C(=NR₁₃)N(R)₂, -N(R)C(=CHR₁₃)N(R)₂, -OC(O)N(R)₂, - N(R)C(O)OR, -N(OR)C(O)R, -N(OR)S(O)₂R, -N(OR)C(O)OR, -N(OR)C(O)N(R)₂, - N(OR)C(S)N(R)₂, -N(OR)C(=NR₁₃)N(R)₂, -N(OR)C(=CHR₁₃)N(R)₂, -C(=NR₁₃)N(R)₂, - C(=NR₁₃)R, -C(O)N(R)OR and -C(R)N(R)₂C(O)OR, wherein each n is independently an integer of from 1 to 5; R₁₂ is selected from the group consisting of C₃₋₆ carbocyclic group and heterocyclic group; R₁₃ is selected from the group consisting of H, CN, NO₂, C₁₋₆ alkyl, -OR, -S(O)₂R, - S(O)₂N(R)₂, C₂₋₆ alkenyl, C₃₋₆ carbocyclic group and heterocyclic group; each R is independently selected from the group consisting of hydrogen atom, C₁₋₃ alkyl and C₂₋₃ alkenyl; each X is independently selected from the group consisting of F, CI, Br and I, provided that when R₃ is - (CH₂)ₙQ, -(CH₂)ₙCHQR, -CHQR or -CQ(R)₂, (i) if n is 1, 2, 3, 4, or 5, then Q is not -N(R)₂, or (ii) if n is 1 or 2, Q is not 5-, 6- or 7-membered heterocycloalkyl,
each of R₄ to R₇ is independently selected from the group consisting of hydrogen atom, C₁₋₃ alkyl and C₂₋₃ alkenyl, and
each of a and b is independently an integer of from 1 to 15.

3. The composition for drug delivery according to claim 2, wherein
each of M₁ and M₂ is independently selected from the group consisting of -C(O)O-, - OC(O)-, -OC(O)-M'-C(O)O-, -C(O)N(R')-, -N(R')C(O)- and -C(O)-, wherein M' and R' are the same as defined in claim 2,
each of R₁ and R₂ is independently selected from the group consisting of C₃₋₂₀ cycloalkyl and C₃₋₂₀ heterocycloalkyl, each of which is independently unsubstituted or substituted with C₁₋₁₈ alkyl or C₂₋₁₈ alkenyl,
R₃ is selected from the group consisting of hydrogen atom, substituted or unsubstituted C₁₋₆ alkyl, and substituted or unsubstituted C₃₋₆ carbocyclic group,
each of R₄ to R₇ is independently hydrogen atom or C₁₋₃ alkyl, and
each of a and b is independently an integer of from 3 to 13.

4. The composition for drug delivery according to claim 3, wherein
each of M₁ and M₂ is independently selected from the group consisting of -C(O)O-, - OC(O)-, -C(O)N(R')- and -N(R')C(O)-, wherein each R' is independently selected from the group consisting of hydrogen atom, C₁₋₁₈ alkyl and C₂₋₁₈ alkenyl,
each of R₁ and R₂ is independently substituted or unsubstituted C₃₋₁₅ cycloalkyl,
R₃ is hydrogen atom, or substituted or unsubstituted C₁₋₃ alkyl,
R₄ to R₇ are hydrogen atom, and
each of a and b is independently an integer of from 5 to 11.

5. The composition for drug delivery according to claim 4, wherein the lipid is one having a structure selected from the following Formulas A to R:
| Formula | Structure | Formula | Structure |
|---|---|---|---|
| A | 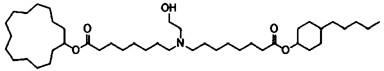 | J | 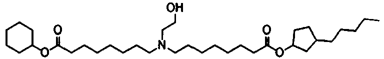 |
| B | 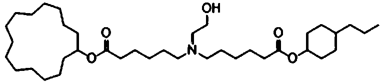 | K | 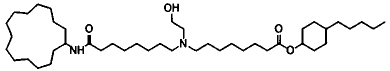 |
| C | 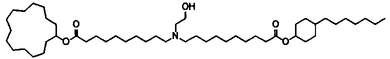 | L | 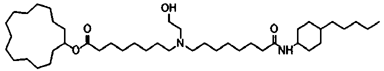 |
| D | 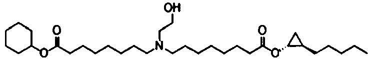 | M | 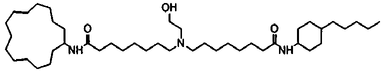 |
| E | 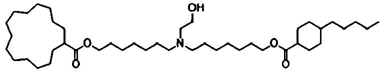 | N | 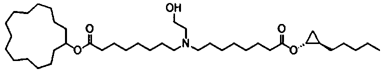 |
| F | 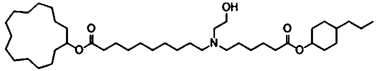 | O | 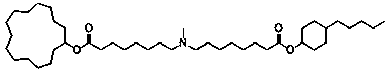 |
| G | 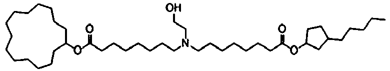 | P | 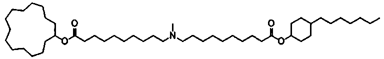 |
| H | 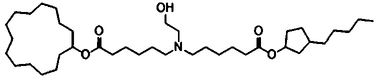 | Q | 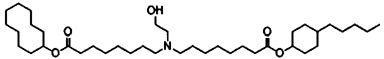 |
| I | 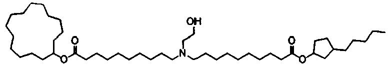 | R | 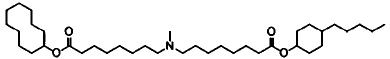 |

6. The composition for drug delivery according to claim 1, wherein the effective ingredient is mRNA.

7. The composition for drug delivery according to claim 1, wherein the lipid-polymer is a polymer in which one or more saturated or unsaturated hydrocarbon groups having 11 to 25 carbon atoms as a hydrophobic part are introduced into a hydrophilic block which is a hydrophilic part.

8. The composition for drug delivery according to claim 7, wherein the hydrophilic block is one or more selected from the group consisting of polyalkylene glycol, polyvinyl alcohol, polyvinyl pyrrolidone, polyacrylamide, and derivatives thereof.

9. The composition for drug delivery according to claim 7, wherein the saturated or unsaturated hydrocarbon group having 11 to 25 carbon atoms is independently selected from the group consisting of lauryl, myristoyl, dimyristoyl, myristyl, palmityl, stearyl, arachidyl, behenyl, lignoceryl, cerotyl, myristoleyl, palmitoleyl, sapienyl, oleyl, linoleyl, arachidonyl, eicosapentaenyl, erucyl, and docosahexaenyl.

10. The composition for drug delivery according to claim 1, wherein the amphiphilic block copolymer is an A-B type block copolymer comprising a hydrophilic A block and a hydrophobic B block, wherein the hydrophilic A block is one or more selected from the group consisting of polyalkylene glycol, polyvinyl alcohol, polyvinyl pyrrolidone, polyacrylamide, and derivatives thereof, and the hydrophobic B block is one or more selected from the group consisting of polyester, polyanhydride, polyamino acid, polyorthoester and polyphosphazine.

11. The composition for drug delivery according to claim 10, wherein the hydroxyl group at the end of the hydrophobic B block is modified with one or more selected from the group consisting of cholesterol, tocopherol, and fatty acids having 10 to 24 carbons.

12. The composition for drug delivery according to any one of claims 1 to 11, wherein the composition further comprises fusogenic lipid.

13. The composition for drug delivery according to claim 12, wherein the fusogenic lipid is one or a combination of two or more selected from the group consisting of phospholipid, cholesterol, and tocopherol.

14. A method for preparing a composition for drug delivery, comprising the steps of:
(a) preparing a solution in which a lipid represented by the following Formula 1; and lipid-polymer, amphiphilic block copolymer, or a mixture thereof; are dissolved in a water-miscible organic solvent; and
(b) to the solution prepared in step (a), adding effective ingredient selected from nucleic acid, polypeptide, virus, or combination thereof, and mixing them: 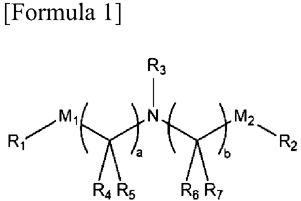 wherein, in the above Formula 1,
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₇ is independently hydrogen atom, or a substituted or unsubstituted, saturated or unsaturated hydrocarbon group, and
each of a and b is independently an integer of from 1 to 20.

15. The method for preparing a composition for drug delivery according to claim 14, wherein the water-miscible organic solvent in step (a) is ethanol.

16. The method for preparing a composition for drug delivery according to claim 14, wherein step (b) comprises:
(b-1) a step of preparing a buffer solution containing the effective ingredient; and
(b-2) a step of adding the buffer solution of the effective ingredient prepared in step (b-1) to the solution prepared in step (a), and mixing them.

17. The method for preparing a composition for drug delivery according to claim 14, wherein step (b) comprises:
(b-1) a step of adding the effective ingredient to the solution prepared in step (a); and
(b-2) a step of adding a buffer solution to the resulting mixture of step (b-1) and mixing them.
